# EUROPEAN PATENT SPECIFICATION

(11) **EP 3 082 679 B1**
(45) Date of publication and mention of the grant of the patent: **04.03.2020**
(21) Application number: 14827931.8
(22) Date of filing: 17.12.2014
(51) Int. Cl.: A61F 13/15

(54) **FLEXIBLE MOUNT CONVERTER FOR FABRICATING ABSORBENT ARTICLES**
WANDLER MIT ELASTISCHER LAGERUNG ZUR HERSTELLUNG SAUGFÄHIGER ARTIKEL
CONVERTISSEUR DE MONTURE SOUPLE POUR FABRIQUER DES ARTICLES ABSORBANTS

(30) Priority: 20.12.2013 US 201361919445 P; 20.12.2013 US 201361919478 P; 20.12.2013 US 201361919502 P; 27.03.2014 US 201461971323 P
(43) Date of publication of application: 26.10.2016
(73) Proprietor: The Procter & Gamble Company, Cincinnati, OH 45202 (US)
(72) Inventor: MACURA, Matthew, Joseph, Cincinnati, Ohio 45202 (US); VANVALKENBURGH, Curtis, Hunter, Cincinnati, Ohio 45202 (US); ESCOBAR, Francisco, Javier, 52076 Aachen (DE)
(74) Representative: P&G Patent Germany
(86) International application number: PCT/US2014/070739
(87) International publication number: WO 2015/095270

(56) References cited:
- EP-B1- 1 251 813
- US-A1- 2011 041 417
- US-A1- 2011 041 997

## Description

### FIELD OF THE INVENTION

The present disclosure relates to a flexible mount converter and methods for manufacturing and/or assembling various components of absorbent articles. The flexible mount converter has improved flexibility, adaptability and re-configurability over current converting lines.

### BACKGROUND OF THE INVENTION

Along an assembly line, various types of articles, for example sanitary napkins, pantiliners, incontinence articles, diapers, and other absorbent articles, may be assembled by adding components to and/or otherwise modifying an advancing, continuous web of material. For example, in some processes, advancing webs of material are combined with other advancing webs of material. In other examples, individual components created from advancing webs of material are combined with advancing webs of material, which in turn, are then combined with other advancing webs of material. In some cases, individual components created from an advancing web or webs are combined with other individual components created from other advancing web or webs. Webs of material and component parts used to manufacture diapers may include: backsheets, topsheets, leg cuffs, waist bands, absorbent core components, front and/or back ears, fastening components, and various types of elastic webs and components such as leg elastics, barrier leg cuff elastics, stretch side panels, and waist elastics. Webs of material and component parts used to manufacture feminine hygiene articles may include: backsheets, topsheets, secondary topsheets, absorbent core components, release paper wrappers, and the like. Once the desired component parts are assembled, the advancing web(s) and component parts are subjected to a final knife cut to separate the web(s) into discrete articles.

US2011/041417, published on February 24th 2011, discloses converting lines for manufacturing absorbent articles, and more particularly, to converting lines having modular process equipment modules and converting mechanisms reconfigurable for making different absorbent articles.

Current concepts around modularity related to high-speed web converting include the concept of 'modules' wherein discrete frame and drive modules are created and installed linearly in series to create consecutive transformations starting from initial web infeed and ending with a final cutter (for example). Typically, these modules are about 1.0m, 1.5m, 2.0m or 2.5m in length. Each module can be operated stand-alone (e.g., as a test stand) and may be mixed and matched with other modules to piece a converter (and desired product) together. The module concept was designed to enable innovation to occur by removing certain modules and installing other modules or by moving module orientations around.

In existing converters, unit operations are limited to available space and must stay well away from the ends of modules where vertical posts and operator stations block available space. Unit operations (aka "unit ops") are of many different sizes and are not typically mounted in close proximity to other unit operations. The existing modularity concept is actually overly cumbersome and expensive as an innovation platform as well as artificially slow due to the execution of the non-process/transformation-based backbone fabrication time (and associated high cost). Another challenge with the current module concept is that the process transformations may not be oriented and positioned in the most process-efficient and space-efficient manner to retain the desired modularity. And the backbone (frame, drive components, panels, guarding, etc.) and ancillaries of each module are quite expensive with the typical 1.0m module costing upwards of $100,000 prior to even loading with unit operations.

Accordingly, there is a need for a simple, spatially-effective, and process-efficient means for imparting rotary transformations onto passing high-speed webs. There is a desire for unit operations which are re-configurable and able to be closely spaced with other unit operations to decrease the footprint of a converting line. What if the current modules were to get longer so as to minimize columns, hardware, and constricting module-based components? What if modules were never (or infrequently) removed from the line/converting floor? What if the frame and drive could be designed with a flexible mounting system that enabled unit operations to easily and quickly flow in and flow out as needed for either product SKU changes or new innovations? What if there was a new modular converter which minimized frame and drive hardware while maximizing flexibility and promoting heavy re-use of unit ops and devices? What if the unit operations themselves became the modular backbone of the converter (vs. a separate skeleton framework) such that they could easily be stacked, oriented in close proximity (or far if needed), and used over and over again as standard building blocks of the converter? What if the modular unit operations could be easily repositioned to an ideal location for optimal space usage and optimal process considerations? These are all objects of the present invention; embodiments of the present invention may combine various objects mentioned. A particular embodiment may, but need not, embody every object of the invention.

### SUMMARY OF THE INVENTION

The present disclosure relates to flexible mount converters and more particularly, to apparatuses which have improved flexibility, adaptability, and re-configurability over current converting lines. In some embodiments, a flexible mount converter for fabricating absorbent articles comprises: a machine direction, a cross direction, a drive side, an operator side, a top side, a left end, and a right end. The flexible mount converter further comprises a length in the machine direction and a width in the cross direction; a drive side and an operator side; a base supporting a horizontal rail mount system; two or more modular unit operations removeably mountable to the horizontal rail mount system in a close-coupled manner such that the distance between adjacent modular unit operations is less than 1m; and complete access guarding. The flexible mount converter further comprises a virtual mirror plate formed of a plurality of plates selected from the group consisting of scab plates, barrier plates, or combinations thereof, wherein the virtual mirror plates are supported by vertical and/or horizontal members attached to the flexible mount converter. The flexible mount converter is capable of producing an absorbent article.

### BRIEF DESCRIPTION OF THE DRAWINGS

The following detailed description of specific embodiments of the present invention can be best understood when read in conjunction with the drawings enclosed herewith.
FIG. 1 illustrates an exemplary current (prior art) converting line.
FIG. 2 compares another exemplary current (prior art) converting line with an exemplary new flexible mount converter.
FIG. 3 illustrates a traditional process layout utilizing non-modular unit operations at typical proximities.
FIG. 4A illustrates a first exemplary new process layout utilizing a park bench and close-coupled modular unit operations.
FIG. 4B illustrates a second exemplary new process layout utilizing a park bench and close-coupled modular unit operations.
FIG. 4C illustrates a third exemplary new process layout utilizing a park bench and close-coupled modular unit operations.
FIG. 5 shows an exemplary scab plate located below the park bench.
FIG. 6 illustrates exemplary horizontal members upon which scab plates may be mounted.
FIG. 7 shows an exemplary scab plate located above the park bench.
FIG. 8A illustrates another exemplary flexible mount converter.
FIG. 8B depicts complete access guarding with foldable guard doors in a closed position.
FIG. 8C depicts the guard doors of FIG. 8B in a semi-opened position.
FIG. 8D depicts the guard doors FIG. 8B in a fully-opened position.
FIG. 8E shows alternative complete access guarding doors in a closed state.
FIG. 8F shows the doors of FIG. 8E in an open state.
FIG. 8G shows another alternative complete access guarding door in a closed state.
FIG. 8H shows the door of FIG. 8G in an open state.
FIG. 8I shows another alternative complete access guarding door.
FIG. 9 illustrates a perspective view of an exemplary park bench apparatus.
FIG. 10 depicts a perspective view of a bottom portion of a pedestal.
FIG. 11 depicts a perspective view of a horizontal rail mount system.
FIG. 12 shows a perspective view of a pair of brackets on top rails of a horizontal rail mount system.
FIG. 13 shows a perspective view of exemplary reconfigurable ribs on a horizontal rail segment.
FIG. 14 illustrates an exemplary flexible mount converter comprising a park bench apparatus, a universal drive stand, and a cantilever modular unit operation.
FIG. 15 is a perspective view of an exemplary cantilever modular unit operation. FIG. 16A is a perspective view of an array of two exemplary close-coupled cantilever modular unit operations.
FIG. 16B is a front view of an array of two exemplary close-coupled cantilever modular unit operations, showing the transformation portions.
FIG. 17 is a perspective view of a simple, cantilever modular unit operation with a simple, direct drive.
FIG. 18 is a perspective view of a cantilever modular unit operation comprising a motor mount utilizing a separate drive stand and constant velocity style jackshaft.
FIG. 19 is a perspective view of a cantilever modular unit operation on a park bench.
FIG. 20 is a schematic of an array of frame-enclosed unit operations.
FIG. 21 is a perspective view of an exemplary non-cantilever modular unit operation.
FIG. 22 is a side view of a modular unit operation.
FIG. 23 is a perspective view of another exemplary flexible mount converter.
FIG. 24 is a perspective view of another exemplary flexible mount converter.
FIG. 25 is a perspective view of a portion of a park bench apparatus.
FIG. 26 is a schematic of an exemplary quick-connect valve coupling system.
FIG. 27 is a schematic of an exemplary quick-connect valve coupling system.
FIG. 28 is a schematic of an exemplary quick-connect valve coupling system.
FIG. 29 is a schematic of an exemplary digital proportional valve.
FIG. 30 is a top view of an absorbent article.
FIG. 31 is a cross-sectional view of the absorbent article taken about line 2-2 of FIG. 30.
FIG. 32 is an exploded view of the absorbent article cross section of FIG. 31.

### DETAILED DESCRIPTION OF THE INVENTION

### Definitions

The following term explanations may be useful in understanding the present disclosure:
As used herein, the term "absorbent article" includes disposable articles such as sanitary napkins, pantiliners, tampons, interlabial devices, wound dressings or bandages, diapers, adult incontinence articles, wipes, and the like. Still further, the absorbent members produced by the processes and apparatuses disclosed herein can find utility in other webs such as scouring pads, dry-mop pads (such as SWIFFER® pads), and the like. At least some of such absorbent articles are intended for the absorption of body liquids, such as menses or blood, vaginal discharges, urine, and feces. Wipes may be used to absorb body liquids, or may be used for other purposes, such as for cleaning surfaces. Various absorbent articles described above will typically comprise a liquid pervious topsheet, a liquid impervious backsheet joined to the topsheet, and an absorbent core between the topsheet and backsheet.
As used herein, the term "joined" encompasses configurations whereby an element is directly secured to another element by affixing the element directly to the other element, and configurations whereby an element is indirectly secured to another element by affixing the element to intermediate member(s) which in turn are affixed to the other element.
The term "substrate" is used herein to describe a material which is primarily two-dimensional (in an XY plane) and whose thickness (in a Z direction) is relatively small (i.e. 1/10 or less) in comparison to its length (in an X direction) and width (in a Y direction). Non-limiting examples of substrates include a web, layer or layers of fibrous materials, nonwovens, films and foils such as polymeric films or metallic foils. These materials may be used alone or may comprise two or more layers laminated together (a "composite substrate"). As such, a web is a substrate.
The term "nonwoven" refers herein to a material made from continuous (long) filaments (fibers) and/or discontinuous (short) filaments (fibers) by processes such as spunbonding, meltblowing, carding, and the like. Nonwovens do not have a woven or knitted filament pattern.
The term "machine direction" (MD) is used herein to refer to the direction of material flow through a process. In addition, relative placement and movement of material can be described as flowing in the machine direction through a process from upstream in the process to downstream in the process.
The term "cross direction" (CD) is used herein to refer to a direction that is generally perpendicular to the machine direction.
The term "park bench" refers to an apparatus comprising a rail-mount system which enables close coupling of modular unit operations in order to form a flexible mount converter.
The term "device" as used herein refers to any individual component on a flexible mount converter that serves a specific function, such as servo motors, push buttons, sensors, operator controls, heaters, pneumatic valves and actuators, or the like. One or more devices may be part of a modular unit operation, or one or more devices may be stand alone.
The term "plug-and-play" as used herein, refers to the ability to quickly and easily configure the function or location of devices as needed by the process or converter. A given motor may be used today for an unwind spindle and tomorrow to drive a modular unit operation. Similarly, a given valve may be used today to load/unload a unit op, but tomorrow, the valve may be used to blow dust off of a sensor. In addition, plug-and-play allows us to choose which motor, valve, or device we are using at any given time, for example, by providing available connections to numerous devices.
The term "power" as used herein refers to the main electrical feeders going to a converter and powering devices. Power may be AC and/or DC.
The term "controls" as used herein refers to means for starting, stopping, adjusting set points, controlling end results, or controlling other functional aspects of devices via pneumatics, relays, solid-state relays, logic controllers, devices themselves, human-machine interfaces, or the like. Controls can be located in a panel or on a unit op. Controls can be physical controls or logical controls.
The term "information systems" as used herein refers to methods for storing and retrieving information from the converter or providing information to the converter. Exemplary methods are parameter recipe loads, human-machine interfaces, supervisory control and data acquisition ("SCADA") systems (e.g., meeting spec limits or control limits), line event data systems (e.g., reliability, other statistical data), time-stamped tag data, chart recorders, scope meters, data collection devices, etc.

### Flexible Mount Converter for Manufacturing Absorbent Articles

A flexible mount converter and method according to the present disclosure may be utilized to manufacture and/or assemble various components of absorbent articles such as diapers or the feminine hygiene articles disclosed in further detail below. The flexible mount converter ("FMC") is a high-speed web converting machine having improved flexibility, adaptability, and re-configurability over current converting lines.

In general, the flexible mount converter comprises one or more of the following components: a base, close-coupled modular unit operations (either cantilevered or non-cantilevered), complete access guarding, and power, controls, and information systems. Each of these components is described in further detail herein. The base refers to an apparatus comprising a horizontal rail-mount system which enables close coupling of modular unit operations in order to form a flexible mount converter. In some embodiments, it is as if the modular unit operations are sitting on a park bench, hence the coined phrase "park bench apparatus". Modular unit operations may be placed in nearly any location imaginable on the horizontal rail-mount system to form the flexible mount converter. Modular unit operations may be cantilever or non-cantilever. A cantilever modular unit operation comprises a transformation portion and a drive portion cantilevered from a load-bearing portion. The modular unit operations are preferably standardized, configurable, re-usable, re-configurable, and stackable. The complete access guarding provides protection as well as accessibility to and visibility of the equipment modules and product stream without interference from vertical posts used in typical converting guarding structures. Finally, plug-and-play power, controls, and information systems allow modular unit operations to be quickly configured and reconfigured. The power, controls, and information systems design flexibility and functionality into the FMC.

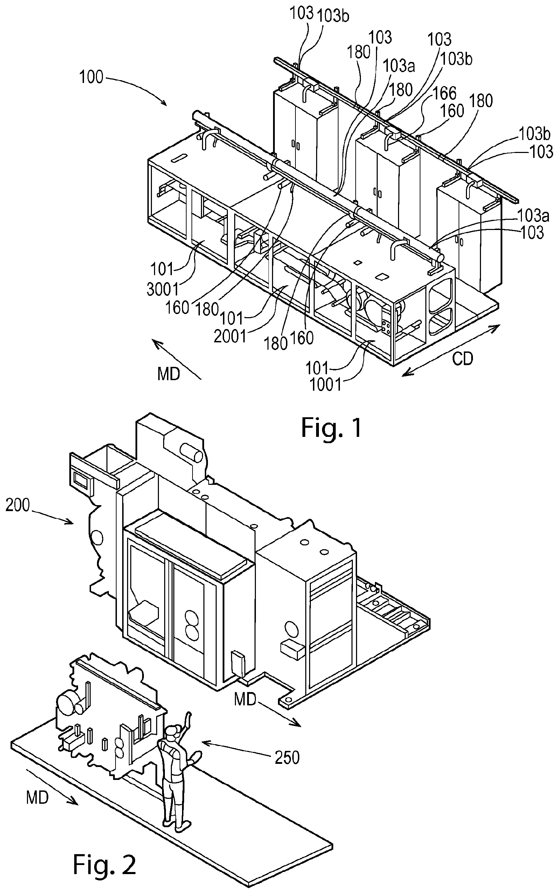

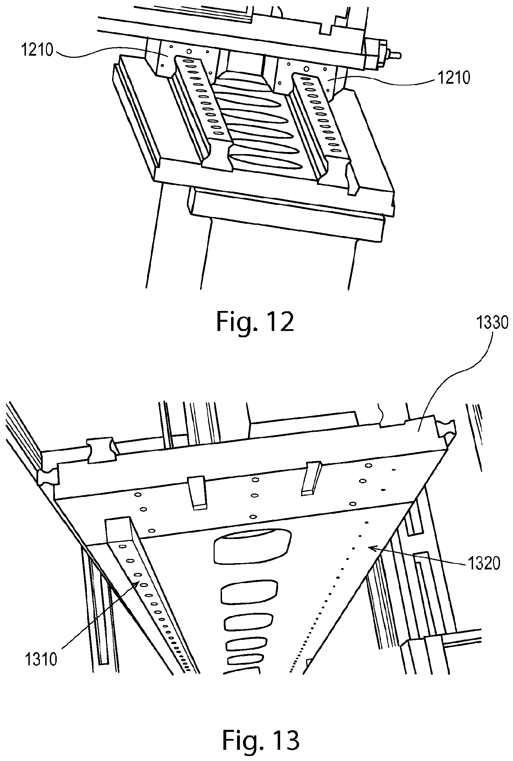

FIG. 1 illustrates an exemplary prior art converting line 100 comprising three converting modules 101 and accessory service modules 103; the converting line 100 is described in US 8,245,384 (see, in particular, Figure 13). FIG. 2 depicts another exemplary prior art converting line 200, along with an exemplary new flexible mount converter 250. While converting line 100 and FMC 250 may have the same or similar number of unit operations, the new FMC 250 is much more compact than the prior art converting line 100.

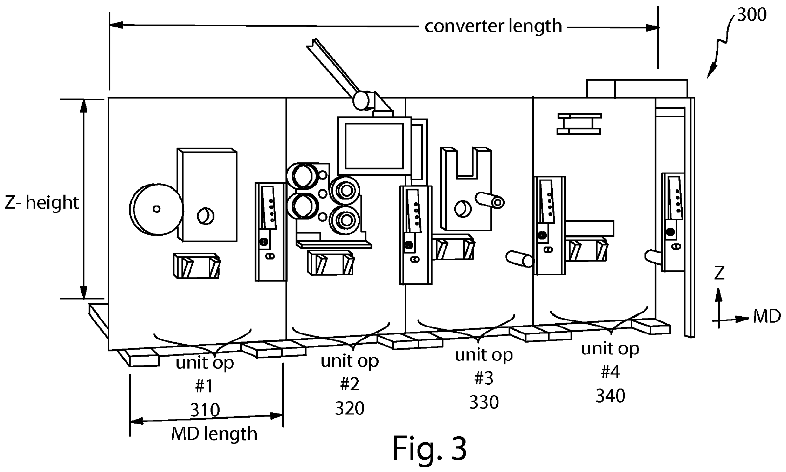

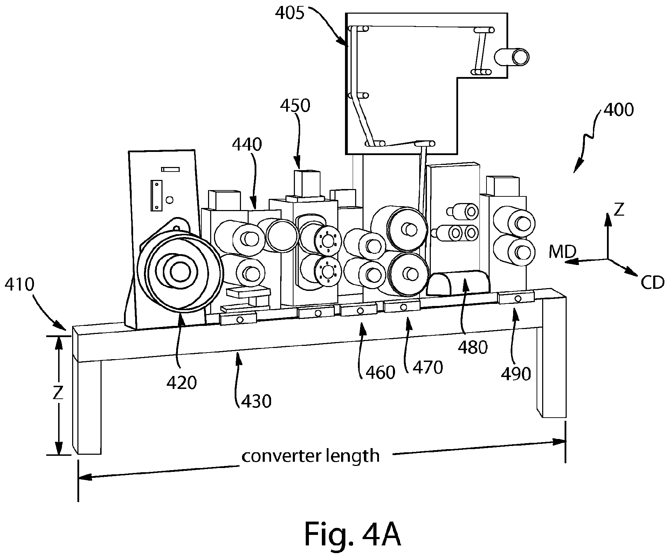

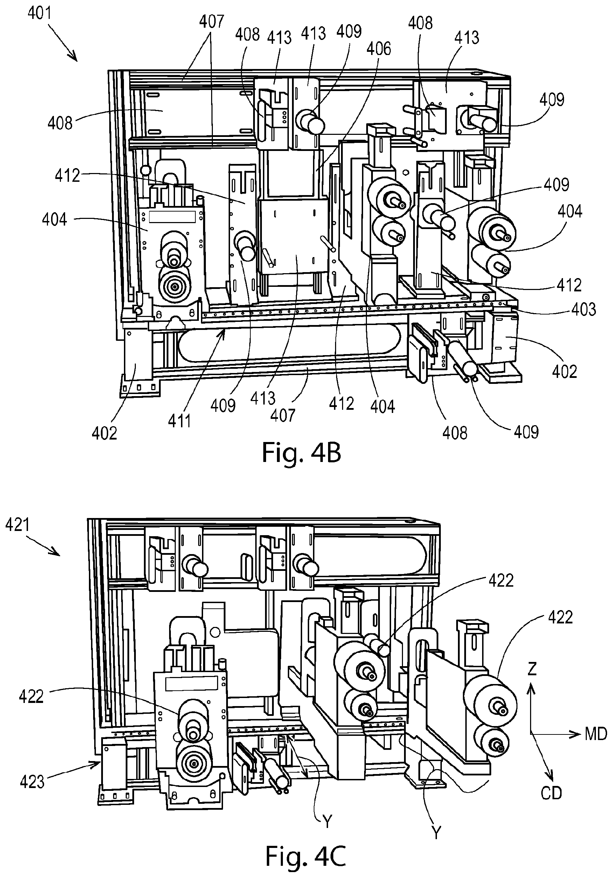

FIG. 3 shows a traditional converter layout 300 utilizing non-modular unit operations 310, 320, 330, 340 in typical layouts. The components of each non-modular unit operation are spread out in the machine direction, cross direction, and z-direction such that each non-modular unit operation has, *e.g.,* a z-direction height, a CD width, an MD length, and a volume. For instance, converter 300 may have a converter length of X (e.g., X = 5m) and comprise Y unit operations (e.g., Y = 4). FIGS. 4A-4C show three exemplary new FMC layouts 400, 401, 421. FIG. 4A shows a FMC 400 utilizing a park bench 410 and close-coupled modular unit operations 420, 430, 440, 450, 460, 470, 480, 490 through each of which a web 405 passes. The term "close coupled" as used herein means that a distance between adjacent modular unit operations (measured from the centerline of one modular unit operation to a centerline of the next adjacent unit operation, each of the centerlines being parallel to a plane extending in the CD direction) is less than 1 m. FIG. 4B shows a FMC 401 having a park bench 411 comprising pedestals 402 and a horizontal rail mount system 403. FMC 401 further comprises close-coupled modular unit operations 404, vertical members 406, horizontal members 407, and devices (web guides 408 and omega rolls 409) mounted on universal drive stands 412 or scab plates 413. FIG. 4C shows an exemplary FMC 421 having a plurality of modular unit operations 422 sitting on a base 423. The unit operations here hang over the front/operator side of the base a distance Y in the cross direction. The distance Y may be greater than or equal to half the width of the modular unit operation, or greater than or equal to the entire width of the modular unit operation.

A converter may have a converter length of .5X (*e.g.,* X = 2.5m) and comprise 2Y modular unit operations (e.g., 8). In this example, converter 400 comprises a unit operation density capacity which is four times that of the traditional converter 300 (four unit ops vs. 16 modular unit operations within the same converter length). While the proportions may vary, it is evident that the FMC may comprise more unit operations (e.g., two times, three times, four times, five times, or more) per converter length than a traditional converter. A 5 m long converter may comprise more than 8, more than 12, more than 16, or more than 20 modular unit operations.

Likewise, the FMC may be shorter in MD length, narrower in CD width, and smaller in volume than a traditional converter having the same general functionality. A converter may comprise one or more modules, and a module may comprise one or more unit operations or MUOs. So, for instance, instead of having a converting line with twenty 1 m-modules (*i.e.*, 20 m total length), the new FMC may comprise one to five 5 m-modules (*i.e.,* 5m to 25m total length) or more. Having four 5 m-modules as opposed to twenty 1 m-modules will result in less restrictive architecture (assuming that for any given module, there is a certain amount of requisite structural architecture, such as columns, etc. every 1-2 m of converter length). For example, rather than having columns every 1-2 m, the new FMC is designed such that the accessible area in a vertical plane is improved versus a traditional converter for instance, stretching the distance between columns to greater than 2 m, 3 m, 4 m, 5 m, or beyond. Similarly, the accessible area in a horizontal plane below the MUOs is much improved over traditional converters.

The FMC may run at a line speed of greater than 500 ft/min, greater than 1000 ft/min, greater than 1200 ft/min, greater than 1400 ft/min, greater than 1600 ft/min, greater than 1800 ft/min, greater than 2000 ft/min, greater than 2200 ft/min, or greater than 2400 ft/min. The FMC may enable very fast line speeds of upwards of 2400 ft/min (roughly 27.4 mph) via the components described herein. For instance, if the modular unit operations are close coupled, the transfer time between unit operations is minimized because transfers may happen at the roll. For example, in some embodiments, the transfer time between adjacent unit operations can be less than about 0.08 seconds (assuming a 12 m/sec web speed); less than about 0.10 seconds (assuming a 10 m/sec web speed); less than about 0.125 seconds (assuming an 8 m/s web speed); less than about 0.166 seconds (assuming a 6 m/s web speed); or any ranges created by these values or any values within these ranges.

The flexible mount converter may enable capital cost reductions of upwards of 25%, or 50%, or 75% compared to a current converter. Likewise, the FMC may enable floor space reductions of upwards of 25%, or 50%, or 75% compared to a current converter. The FMC will introduce flexibility for future innovation such that a significant reduction in cost, time, and effort is enabled for re-tool and changes to enable future process and product configurations. The platform is to be re-configurable by re-arranging/re-tooling modular unit operations vs. the current approach of re-arranging and re-supplying new modules (frame, baseplate, mirror plate, drive, etc.) and new unit operations. This flexibility will allow for an array of different products to be made on one converting line; for instance, different types of sanitary napkins, incontinence articles, and pantiliners (having varying features selected from the group consisting of thick, thin, winged, non-winged, big, small, wrapped, folded, shaped chassis, three-layer, seven-layer, colored, printed, bonded, embossed, combinations thereof, etc.) may all be made on the same general converting line just by reconfiguring modular unit operations between runs.

The flexible mount converter can provide improved accessibility (*e.g.*, for operating, cleaning, and maintaining) over current converter designs. The FMC can enable increased throughput (e.g., design for 3,000 pads per minute or 4,000 pads per minute, etc.). The FMC can be designed for ease of obsolescence; for instance, as PC&IS (power, controls, and information systems) hardware and capability evolves, the ideal flexible mount converter will enable evolution through managed upgrades in existing enclosures/panels versus large re-designs and replacements. The FMC may maximize the use of affordable automation to further drive down manufacturing & operating expenses and general overhead costs. The FMC may be designed to reduce or eliminate noise generators where feasible. Similarly, the FMC may be designed to reduce air usage (pneumatic and vacuum/dust) where feasible. Importantly, the FMC is designed for improved sustainability via a reduction in energy consumption and material usage.

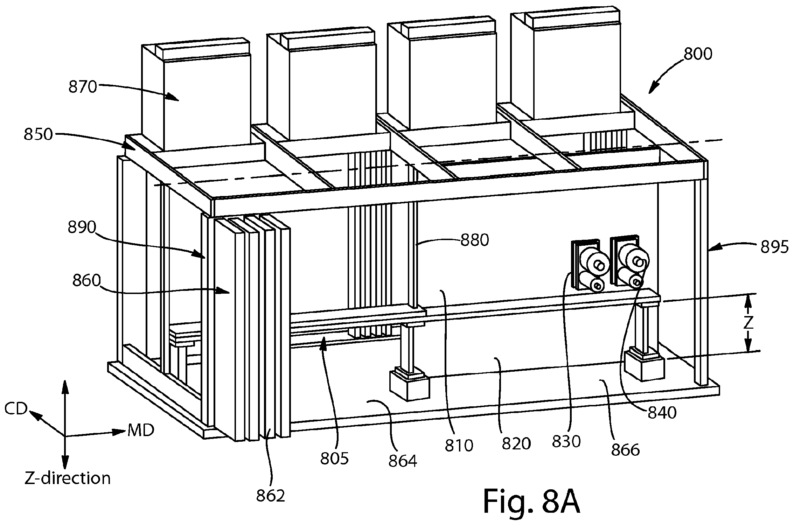

The flexible mount converter comprises a machine direction, a cross direction, and a vertical z-direction. The FMC typically comprises a combination of the following components: one or more park benches, two or more modular unit operations ("MUOs"), virtual mirror plates, guarding, and related power, controls, and information systems to enable successful operation. These components are shown in FIG. 8A and described in further detail below. In general, the park bench involves a rail mount system that enables close coupling of MUOs in nearly any location imaginable in order to assemble units and form the FMC.

Exemplary converting lines that could benefit from the FMC and associated components described herein include U.S. provisional application serial nos. 61/918,670 and 61/918,671, both filed on December 20, 2013.

### Virtual Mirror Plate for a Flexible Mount Converter

The FMC comprise a virtual mirror plate. The virtual mirror plate concept comprises employing re-configurable small plates that can be utilized to support devices both above and below the park bench. The plates separate the operator side and drive side, mainly to separate sources of contamination from the product. It is a "virtual mirror plate" because it is not a traditional, full/large, aluminum 20 mm plate which covers a significant portion above of the converting line vacant space. The virtual mirror plate ("VMP") consists of a plurality of small "scab plates" which are used to mount or surround individual devices such as metering rolls or tracking tables and/or a plurality of small "barrier plates" which are used to fill in the gaps between scab plates to more completely separate the operator side from the drive side. The plates are cheaper, easier to install/remove, and enable faster re-configuration of devices above and below the park bench than a traditional, large, one-piece, non-reusable mirror plate.

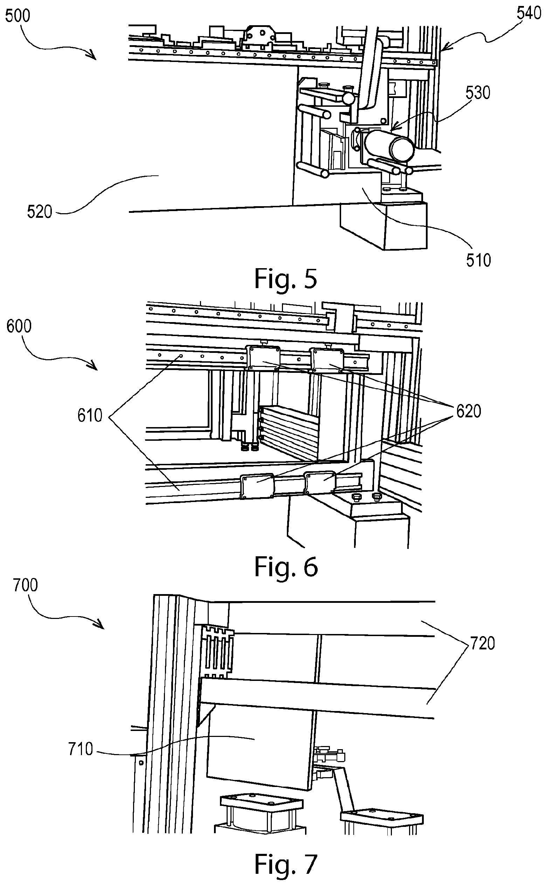

FIG. 5 shows a portion of a converter 500 having both a scab plate 510 and a barrier plate 520 located below a base 540 (park bench in this particular embodiment). A device (e.g., metering roll or idler) 530 is mounted on the scab plate 510. The plates are supported by vertical and/or horizontal members above and/or below the park bench (e.g., extending from the converter frame) which form a framework for adding plates as needed. Extruded aluminum members are preferred because they are highly reconfigurable versus welding a frame. Devices (e.g., printers, vision systems, driven rolls, web guides, metering rolls, idlers, etc.) may be attached to a vertical or horizontal member with or without the use of a plate, as long as the devices are not too heavy for the supporting member.

FIG. 6 illustrates a portion of a converter 600 having two exemplary horizontal members 610 upon which plates may be mounted, as well as four carriages 620. The carriages are on rollers, so any mounted plate is slideable. Scab plates are preferably made of aluminum. Barrier plates may be made of plastic (and are preferably white (or light) in color to denote cleanliness and simplification). Aluminum or other materials would also work for the barrier plates, however, these options may be more expensive and less reconfigurable such that it is easier to throw them out and start over than it is to simply change them. The scab and barrier plates work as barriers to keep the FMC operator side in accordance with good manufacturing practices ("GMPs") reduce contamination, etc.

FIG. 7 shows a portion of a converter 700 from the drive-side, showing an exemplary scab plate 710 mounted on horizontal members 720 located above the park bench. There may be both an upper virtual mirror plate 810 and a lower virtual mirror plate 820, wherein each VMP is made up of a plurality of plates, such as shown in FIG. 8A. VMPs are highly reconfigurable with minimal effort and cost because they don't need to be re-machined, re-welded, re-painted, etc. A full mirror plate (made of plastic or aluminum) will be added surrounding the scab plates only if deemed necessary for good manufacturing practice (*e.g.,* to remove sources of contamination from product), for sound abatement, or the like. Re-configurable VMP assemblies may be sent to plants as kits, for instance, comprising extruded aluminum, scab plates, and barrier plates. For example, a converter having a 350 mm centerline may be supplied with a kit to set up VMPs to a 250 mm centerline (or to any desired centerline in between). The converter design is such that any MUO/device with a centerline between about 100 mm and 500 mm, or between about 250 mm and 350 mm can be used at the same time on the same converter via the use of CD spacers. It is to be appreciated that the converter centerline may vary within the same converter, or from converter to converter.

### Complete Access Guarding System for a Flexible Mount Converter

The new guarding for the FMC is referred to as a "complete access guarding" ("CAG") system and is associated with the FMC. Specifically, the CAG may be desired for safety, noise abatement, or the like. FIG. 8A illustrate a flexible mount converter 800 comprising a park bench apparatus 805, virtual mirror plates 810, 820 and MUOs 830, 840 with a surrounding frame 850, complete access guarding 860, and panels 870. The frame for the FMC generally stays on the line as MUOs are switched in and out. The frame may comprise minimal infrastructure. Re-configurable extrusion-based guarding may be used to enclose the converter. The FMC may be enclosed in repeatable CAG frame segments; for example, two 2.5 m park benches may be housed within one 5.0 m CAG. The frame may be removably attached to the base or integrally formed therewith. The CAG may be movably connected to the frame as described below.

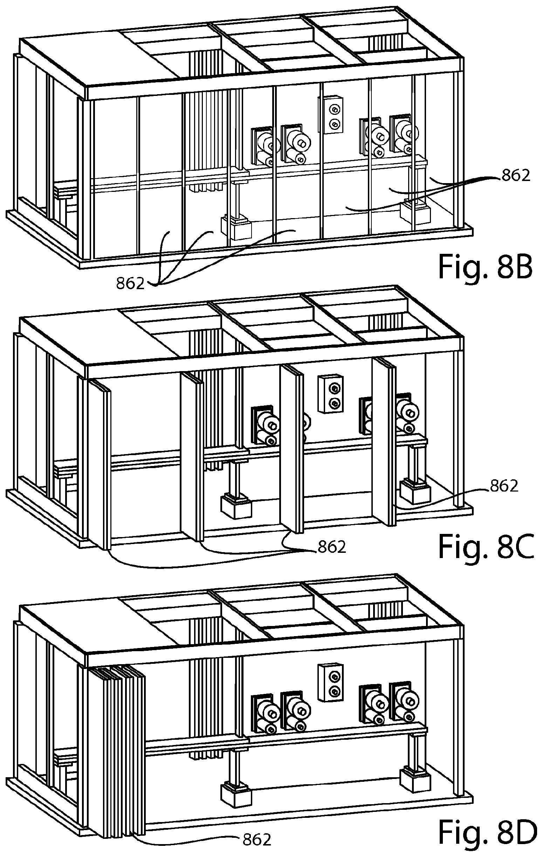

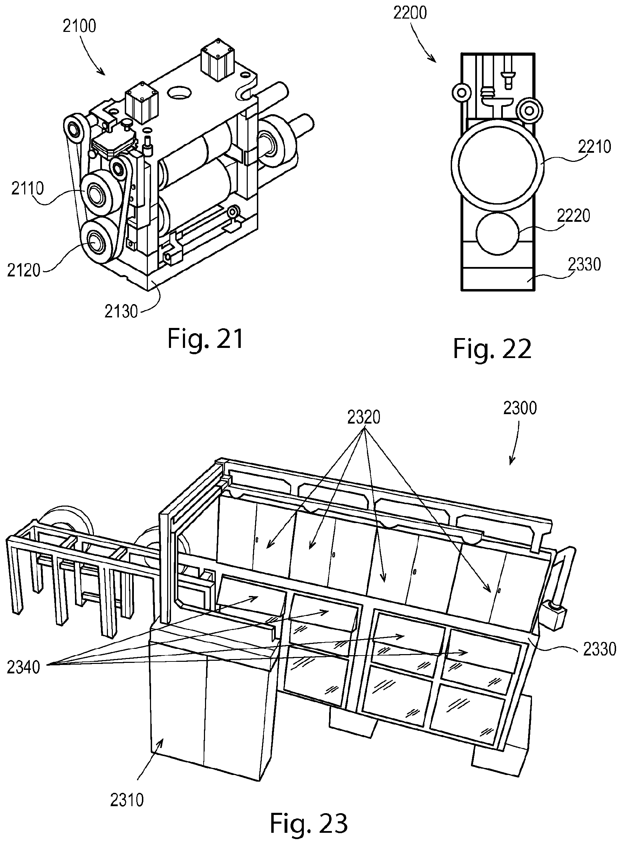

In some embodiments, the FMC is at least partially enclosed in a frame 2320 (shown in Figure 23). The CAG may be operatively connected to the fram 2320. For example, in some embodiments, the CAG may be slidably connected to the frame 2320 or may be pivotally connected to the frame 2302. Still in other embodiments, the CAG may be slidingly and pivotally connected to the frame 2320 (see Figure 8D).

Preferably the FMC does not comprise vertical posts/columns which block available space. If there must be columns 880 *(e.g.,* for structural or emergency stop reasons), they're preferably centered on the park bench in the machine direction (vs. in a plane parallel to the park bench) so they do not block access to MUOs. It may be desirable to have an emergency stops located on vertical posts at the start of the converting line, or every 5 m along the length of a converting line, or at other convenient locations along the line such that operators can stop the line as needed without traveling long distances. The CAG described herein enables full operator side and drive side access to MUOs for maintenance, changeovers, and the like. Similarly, the FMC described herein enables full operator side and drive side access to MUOs because the FMC comprises no permanent mirror or back plate to hinder access. Optionally, the FMC may comprise a removeable, non-permanent mirror plate.

As stated above, guarding around a converter may be desired for safety, noise abatement, or the like. However, this guarding is often cumbersome and provides limited access to the converting line. The new guarding 860, shown in FIG. 8A, provides complete access to unit operations for cleaning, maintenance, and line re-configuring. CAG may be provided on the operator side, drive side, or both sides of the FMC. In some embodiments, the CAG may additionally be located on the top side, bottom side, or ends of the FMC. The guarding assembly is broken into repeat lengths based on the park bench length (e.g., 5 m). Vertical posts on the front of the converter are spaced to intervals such that the CAG may be post-less along the majority of the converter (e.g., the CAG may comprise zero vertical posts within a 5 m-long segment). For instance, there may be an open-access area or post-less span which is greater than about 2.5 m, or greater than about 3 m, or greater than about 4 m, or greater than about 5 m, or greater than about 6 m in length in the MD. This open-access area may be present on the drive side, the operator side, or both sides of the FMC.

In a preferred embodiment, the CAG comprises a series of foldable guard doors 862 on a track 864 (akin to bi-fold, accordion-style closet doors having an upper and lower track). The doors can slide anywhere within this e.g., 5 m length/range; the doors may slide partially or completely out of the way towards either the left end 890 or right end 895 of the converter. FIG. 8B depicts foldable guard doors 862 in a closed position; FIG. 8C depicts the guard doors 862 in a semi-opened position; and FIG. 8D depicts the guard doors 862 in a fully-opened position at the left side 890 of the converter. The guard doors are capable of sliding out of the way (of the portion of the FMC of interest, for example, a particular MUO or array of MUOs) such that full FMC length (*e.g.,* 5 m) has zero vertical guard posts and zero structure on the floor thereby enabling full access to converter anywhere for maintenance and changes. A horizontal track is located at about ground level - meaning that the track may be at ground level or slightly raised above or sunken below the actual ground level. For instance, the lower door track 864 is in the floor slab 866 and covered such that only a narrow slot interrupts the continuous floor surface. Preferably, the track is located in the floor slab such that it is not raised and thus does not present a tripping or safety hazard. Additionally, a flush or sunken track will not impede equipment which must be wheeled in. The CAG may include a top portion, such as a clear polycarbonate guard.

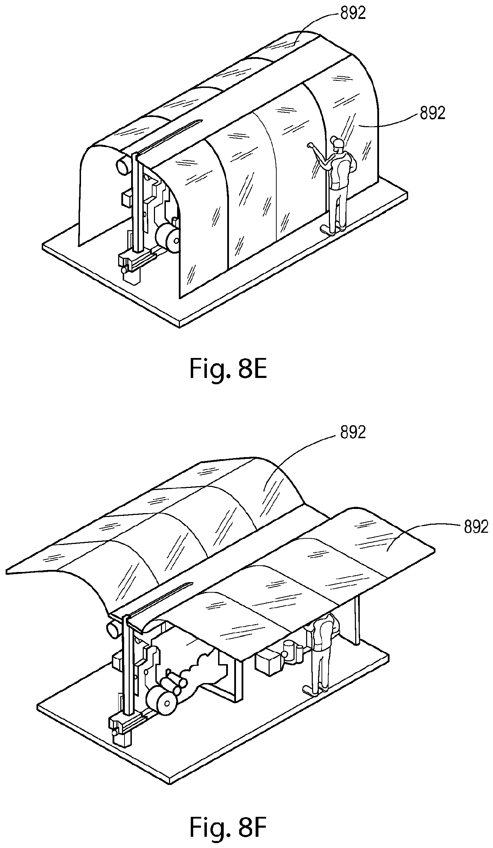

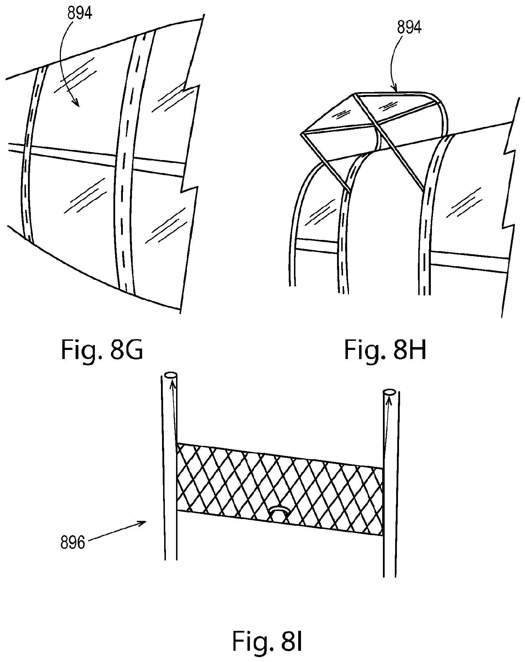

Alternatives to the foldable guard doors include the use of accordion folding doors (doesn't need a vertical track; the doors hinge and accordion against one another in an alternating fashion); gullwing style doors (don't need a vertical track); fold-up doors (needs a vertical track); vertical sliding doors (needs a vertical track); roll-up garage doors (needs a vertical track; can roll up into a circle); sliding (but not-folding) doors that slide vertically, horizontally, or both; or standard vertical pull-open hinged doors. One or more of these types of guarding may be used to make up the complete access guarding system. Exemplary gullwing doors 892 are shown in a closed position in FIG. 8E and an open position in FIG. 8F. An exemplary fold-up door 894 is shown in a closed position in FIG. 8G and an open position in FIG. 8H. An exemplary vertical sliding door 896 is shown in FIG. 8I.

Panels, such as electrical panels and drive panels, may be located on the top and/or rear portions of the frame to increase access or open area around the MUOs or decrease the footprint of the converter. In one embodiment, electrical panels are located on the top, drive side of the guarding. This enables significant spacing on the plant floor without building a complete mezzanine support system. The FMC may comprise a plurality of elevated panels along with a main power distribution, safety inputs/outputs, and central programmable logic controller in a single floor-mounted panel. In one embodiment, each drive panel comprises four large drives (*e.g.,* for MUOs) and four small drives (*e.g.,* for devices).

Keyless (*e.g*., magnetic) guard door switches may be employed to prevent access to moving equipment on the converter. Keyless switches may be less susceptible to breakage due to improper alignment or hard closures. Magnetic switches enable easier alignment between switch actuator and target. Guard switches can be connected to Ethernet communication devices for diagnostics and monitoring of each switch with the logic controller.

### Base for a Flexible Mount Converter

The FMC of the present invention should be provided with a sturdy base. The sturdy base can help eliminate or at least reduce vibration in the FMC and/or MUOs caused by operating speed of the MUOs. The sturdy base should provide the FMC with the flexibility of having MUOs being releasably attached and/or slidably attached thereto allowing for flexibility in the configuration of the MUOs.

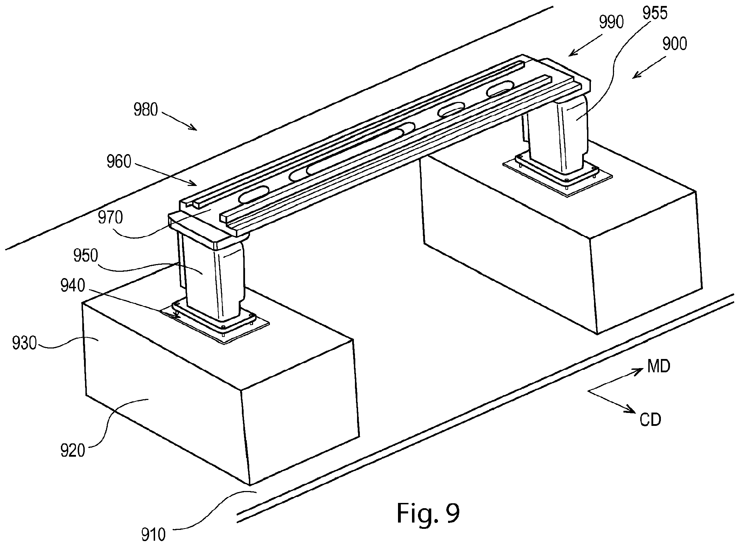

In some embodiments, a "park bench" apparatus shown in FIGS. 9-14 (among others) can be utilized as the base with the flexible mount converter discussed above. The park bench minimizes materials, change effort, and cost while maximizing flexibility in placement, adjustment, and re-use of MUOs. The park bench involves an adequately sturdy, horizontal rail mount system that enables placement of a plurality of modular unit operations ("MUOs") in nearly any location imaginable to form the flexible mount converter ("FMC"). In one embodiment, the park bench enables MUOs to float on the park bench. By "float" it is meant that the MUO does not substantially touch or rest on the ground; rather, the MUO is supported by the park bench such that the MUO is raised a distance Z from the ground (as shown in, *e.g.,* FIGS. 4A, 8A, 9).

In some embodiments, distance Z can be any reasonable distance. Distance Z can be from greater than about 1 mm to about 1700 mm, from about 100 mm to about 1500 mm, from about 200 mm to about 1200 mm, from about 400 mm to about 1000 mm, from about 700 mm to about 900 mm, or any values within these ranges or any ranges created by these values. The concept of floating unit operations maximizes process/transformation efficiency and operability. In some embodiments, the distance Z may correspond to the height of the horizontal rail segment disclosed hereafter.

MUOs may be constructed on or connected to the park bench so as to operate and/or adjust smoothly. The park bench enables simple and easy re-configuration of MUOs with minimal time, cost, and re-investment. The concept minimizes mechanical/structural components (*e.g.*, columns, hardware, and constricting module-based components) and uses a simple mount method to enable MUOs to quickly be moved and web paths re-configured as needed for product changes and future innovations with minimal time, effort, and cost. MUOs may more easily be placed on and taken off the park bench (*e.g.,* due to accessibility of all sides of an MUO, including the underside since it is raised a distance Z from the ground) compared to the ordeal of repositioning unit ops on traditional converter (*e.g.,* due to a non-modular, complex shape which makes it hard to remove or reposition). MUOs may also be capable of sliding along the horizontal rail to a different location along the length of the park bench.

By utilizing the park bench concept to locate MUOs on a centerline oriented in the machine direction of the FMC and utilizing spacers and pedestals to change orientation and heights, the MUOs can be located anywhere required and in any proximity to optimize space and process. While the park bench is described with reference to MUOs, it is to be appreciated that while the inventive MUOs were specifically designed with the park bench in mind, there may be other unit operations that can be configured to sit upon the park bench. The park bench concept allows for tune-able features either in a manual, semi-automatic, or fully automated manner (e.g., push-button change-over).

In one embodiment, the park bench apparatus comprises two or more vertical supports (aka "pedestals") and a horizontal rail mount system comprising one or more horizontal rail segments. FIG. 9 illustrates an exemplary park bench apparatus 900 comprising a floor slab 910, rebar 920, concrete 930, two footers 940, two pedestals 950, 955, a horizontal rail mount system 960, and one horizontal rail segment 970. The park bench 900 has a first end 980 and a second end 990. A park bench is like a module, wherein one or more park benches may be used to create a FMC; typically the first end of a first park bench is joined to the second end of a second park bench in series. The apparatus depicted in FIG. 9 is a single unit which is 2.5m in length; a FMC may comprise two of these units (comprising three pedestals and two horizontal rail segments), thus creating a 5m converting line. The horizontal rail mount system 960 typically runs substantially the entire length of the park bench, from a first pedestal 950 to a second pedestal 955; however, there may be embodiments wherein the rail mount system is longer than the park bench. Each horizontal rail segment may be from about 0.5 m to about 6 m, or from about 1m to about 5 m, or from about 2 m to about 4 m, or about 2.5 m long. As described above, and shown, *e.g.,* in FIGS. 8A-8I, the park bench may be surrounded by a frame comprising complete access guarding and electrical panels.

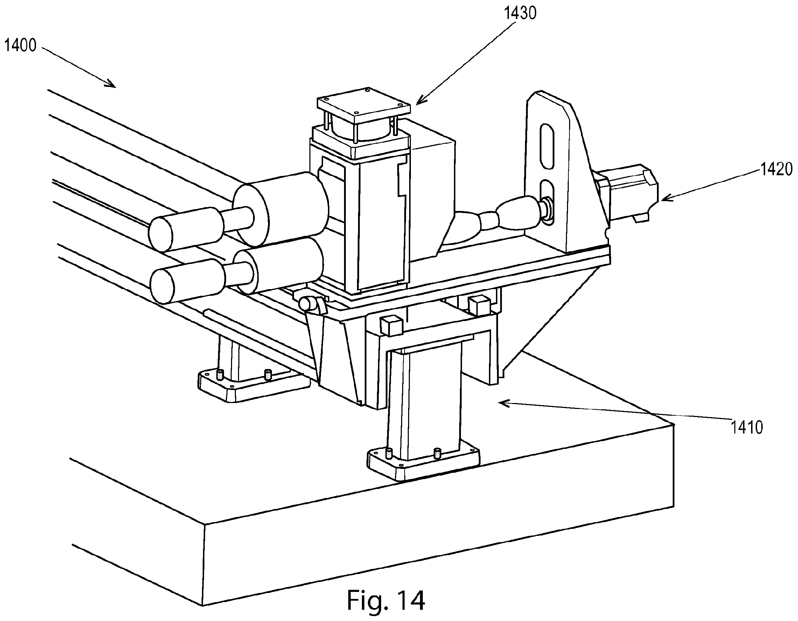

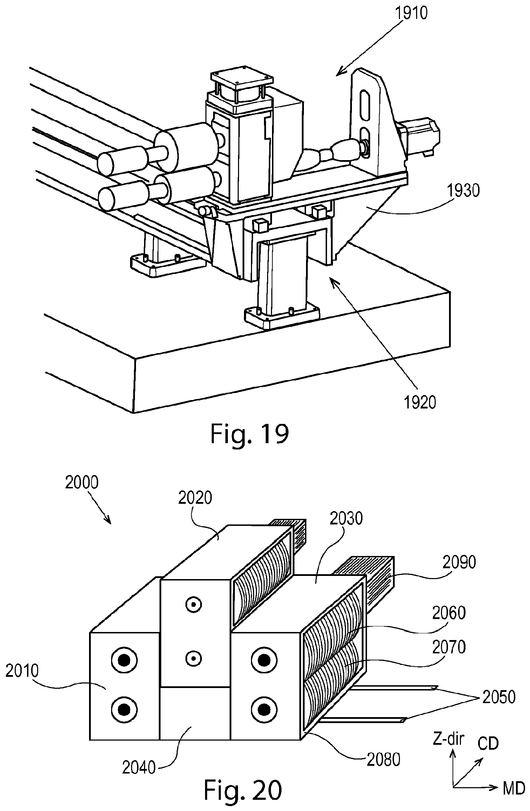

The pedestals are "sharable" such that they are each capable of supporting the ends of two horizontal rail segments at a time. A pedestal can support just one horizontal rail segment, such as when only one segment is used on a converter. Or, a single pedestal may support four or more horizontal rail segments, for instance, if a north-south converting line and an east-west converting line cross paths via an intersection at the pedestal. Most commonly, the park bench apparatus will comprise at least three pedestals and at least two horizontal rail segments aligned in the MD. A pedestal can be mounted to fully support the horizontal rail segment (if farthest upstream/downstream park bench), or the horizontal rail segment may only be mounted onto a first half of the pedestal, wherein a second horizontal rail segment may be mounted to the second half of the pedestal, such that the pedestal supports both rail segments. In other embodiments, pedestals need not be sharable; they may be dedicated to a single rail segment, such as shown in FIGS. 14 and 19.

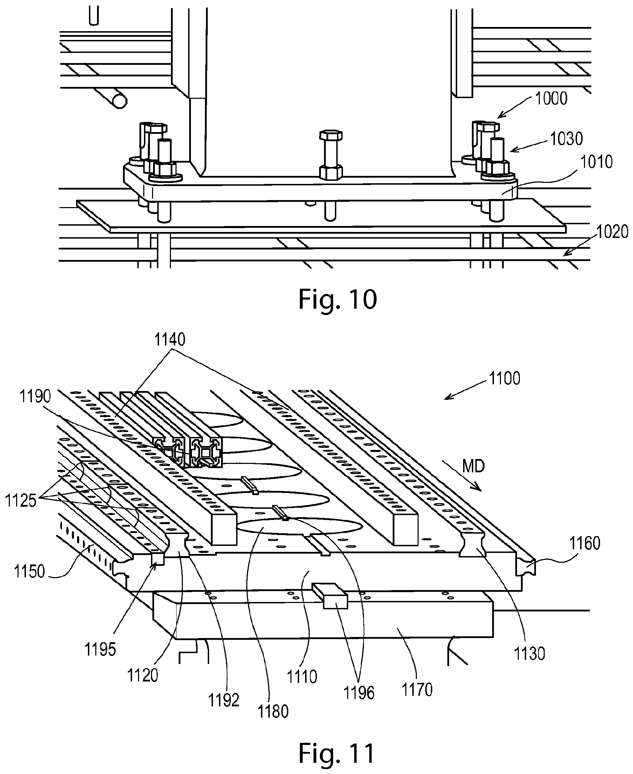

FIG. 10 depicts a perspective view of a bottom portion 1010 of a pedestal 1000. The pedestals are secured in foundation footers 1020 the ground under the pedestals. The use of footers makes the FMC a more permanent structure than a current converter; however, the FMC is more customizable. The footer design minimizes converter infrastructure while minimizing converter installation costs. Footers are intended to fully support all loads (static, dynamic, vibrational) transmitted from MUOs, in and through the park bench, and into the pedestal. The concept intent of the footer can be summarized as follows: minimize the mass, structure, complexity, and cost of the converter structure by supporting all loads directly on concrete/rebar in the ground and isolating vibration of the converter from the floor slab. The belief is that cost can be driven out of the converter infrastructure (frame, base, mirror plate, drive, ancillaries, etc.) by increasing the loaded/installation costs to result in a cheaper (more cost-effective) converter that is installed once and not moved/re-located (e.g., has a useful life of at least 10-15 years). Footers may be poured/installed in the ground; footers may comprise cast-in J-Hook anchors, epoxy anchors, or the like that will be firmly located in the concrete for securing the pedestal to concrete.

Pedestals may be from about 0.1 m to about 1 m, or from about 0.25 m to about 0.75 m high. The height of each pedestal is adjustable. Vertical (height adjust) of the pedestal/park bench will be executed via adjustable jacking bolts 1030. This method is common in the industry. There exists a jacking plate that sits on top of the concrete with through holes for the anchors which pull the pedestal downwards and no through-holes for the jacking bolts that push the pedestal upwards (enables fine tune vertical position and angle adjustment). Pedestals are also adjustable in the machine direction and cross direction. CD and MD alignment can be completed strictly by using oversize holes in the pedestal and moving the pedestal until precise alignment is achieved. Oversize washers can be tack-welded in position on the oversize holes in order to preserve alignment (align and lock at assembly).

FIG. 11 depicts a perspective view of an embodiment of the horizontal rail mount system 1100. The horizontal rail mount system is like a counter upon which MUOs are mounted. The horizontal rail mount system comprises a horizontal rail segment 1110, or bench, comprising one or more mounting rails 1120, 1130, 1140, 1150, 1160. A plurality of horizontal rail segments may be used in combination with pedestals 1170 to form the park bench. Rail segments may be added or subtracted as needed to meet the desired machine direction converter length. The horizontal rail mount system enables close coupling of MUOs for close packed /compact converter design. In a preferred embodiment, the rail system comprises a carriage ball-bearing system for easy movement of heavy unit operations.

As shown, the mounting rails 1120, 1130, 1140, 1150, and 1160 may comprise a plurality of openings allowing positional placement of the MUO's, mirror plates, scab plates, etc. as desired. Additionally, unlike conventional converters, the FMC of the present invention provided for facilitates mounting of MUOs, mirror plates, scab plates etc. off of the front and/or the back of the FMC via the mounting rail 1150 and 1160. In contrast, in conventional converters, components desired to be placed on the front and/or back of the converter would have required drilling into the converter or a portion thereof.

Mounting rails may be located on the top, front, and/or backside of the rail segment to enable easy mounting of MUOs and devices (*e.g.*, process delivery devices) while also enabling movement of devices in either a manual or even in an automated manner. MUOs may be mounted anywhere in the machine direction along the horizontal rail. Mounting rails may comprise a plurality of shorter rail sections 1125 such that one or more sections may be removed (and later re-installed) in order to more quickly and easily add or remove MUOs.

The horizontal rail segment is generally positioned from 0.15 m to 1 m, or from 0.3 m to 0.8 m, above the ground. It is preferred that the pedestals and horizontal rails be positioned such that MUOs placed on the rails are easily accessible to operators for maintenance, repairs, etc. There are many additional benefits to having the horizontal rail segment raised a distance from the ground, such as ergonomics, air flow around all sides of the MUOs, minimizing required infrastructure, etc. The vertical height of the rail segment may be minimally adjustable in the z-direction above the height of the pedestals upon which it rests. It is envisioned that the park bench could, instead of being generally waist-high, be much closer to the ground. In some cases, there may be no need for pedestals, such as when the horizontal rail segment is located on (or in) the ground. The horizontal rail segment is generally positioned parallel to the ground (i.e., at a 0 degree angle from the ground). However, it is within the scope of the invention for horizontal rail segments to be at an angle from the ground, such as 5 degrees, 15 degrees, or from 0 degrees to 45 degrees; pedestals may be adjusted accordingly. So, while it promotes standardization and flexibility to create a park bench having uniform height pedestals and horizontal rail segments parallel to the ground, it is also envisioned that pedestals may comprise an assortment of heights and rail segments may comprise varying angles of inclination.

In one embodiment, the horizontal rail segment at least partially comprises ductile cast iron to take advantage of the inherent damping properties of cast iron (significantly more optimal versus wrought/annealed steels). The horizontal rail segment may comprise large holes 1180 to enable ease of wire and pneumatic line routing to the MUOs. The horizontal rail segment may comprise additional spacers 1190 or mounting surfaces 1140.

Embodiments are contemplated where the horizontal rail segment is integrally formed with the base. For example, the horizontal rail segment may be integrally formed with the pedestals. Embodiments are contemplated where the horizontal rail segment is integrally formed with the front rail and / or back rail. Embodiments are contemplated where the horizontal rail segment is integrally formed with the front rail, back rail, mounting rail(s) and/or pedestals. In these embodiments where the horizontal rail segment is integrally formed with another element, it is meant that the horizontal rail segment along with integrally formed elements is formed from one piece of steel.

In other embodiments, the horizontal rail segment may comprise a plurality of portions which are rigidly attached (excluding integral as defined above) to one another via welding, bolting, the like, and/or combinations thereof. Similarly, embodiments are contemplated where the horizontal rail segment is rigidly attached to the pedestals, the front rail, the back rail, and/or the mounting rails.

In one embodiment, the horizontal rail-mount system comprises two parallel top rails a front rail 1120 and a back rail 1130 which enable MD-adjustable positioning of unit operations, metering rolls, devices, etc. The front rail is located against a high toleranced, MD-oriented machined ledge 1192 which then enables location of all features/units, etc. in the converter cross direction. A rail clamp 1195, or wedge, forces the front rail against the high toleranced machined ledge, thereby precisely aligning the front rail. This is an important alignment feature on the converter. The back rail is 'floating', meaning that it is located only by the front rail and a high toleranced fixture between the front and back rails. The front rail is installed first. Then, with the back rail fasteners left loose (or snug), the back rail is precisely located relative to the front rail with a fixture, then the back rail fasteners are tightened. The back rail is precisely 'located at assembly' through this process. Carriages may be joined to each of the front and back rails, such as shown in FIG. 12. A third or additional rail may be located between the front and back rail. Smaller vertical rails may be located on both the front side and the back side of the horizontal rail mount system that are used to assist in supporting cantilever (overhung) loads via gusset style brackets. These may be included for structural purposes.

The horizontal rail-mount system comprises a keyway and key 1196 such that the front face of the keyway and key precisely locate the centerline of the park bench and thereby becomes a reference for alignment anywhere on the converter relative to process centerline. This key way is located with a high tolerance relative to the front rail locating ledge. Mounting surfaces are available for mounting and locating devices. Not all uses for mount surfaces have been predefined, however; these provide flexibility for future mount and location of devices. For example, they may be used to locate metering roll assemblies and keep them locked into position. MD oriented keys/keyways may assist in the cross direction alignment of each park bench assembly. This concept will minimize alignment efforts during installation.

The horizontal rail-mount system additionally comprises mounting clamps (not shown) to mount MUOs on the horizontal rail segment. The clamps can be mechanical, hydraulic, pneumatic, combinations thereof, or the like. For instance, the rail clamp system can either be via a mechanical fastener/handle for manual lock or a pneumatic clamp system for greater clamping force. Preferably, the clamps are located next to carriages 1210, shown in FIG. 12.

The horizontal rail-mount system may further comprise two machined rib-mounting surfaces, one in front and one in back. These ribs are structural to add support; they are not typically used to mount devices like the mounting rails discussed above. This design thereby enables the ribs to be added or modified (re-configured) to adjust the moments of inertia, torsional stiffness, and bending stiffness to ensure the park bench design matches the required stiffness for specific configurations. For example, there is only one (front) rib 1310 on the horizontal rail segment 1330 shown in Figure 13 which is designed to create an unequal bending stiffness, front to back, and offset the significant overhung loading for worse case scenarios when the maximum number of MUOs are mounted on the park bench. The front rib only design ensures that deflections in the front of the park bench are near equal (within 0.010 mm) of deflections in the back of the park bench, thereby ensuring the mounting rails remain level within an acceptable tolerance. Rib-mounting surface 1320 is unused in this example.

The horizontal rail-mount system may comprise universal drive stands which provide full flexibility to mount MUOs anywhere on the park bench in the machine direction. FIG. 14 illustrates an exemplary flexible mount converter 1400 comprising a park bench apparatus 1410, a universal drive stand 1420, and a cantilever modular unit operation 1430. A family of universal drive stands may enable mounting of MUOs on a desired center or centerline. As previously mentioned, spacers may be used to locate MUOs to correct heights as needed by the process layout. Universal drive stands may be designed to accommodate MUOs comprising tooling such as ring rolls, knives (e.g., die cutting or other means), bond/crimp/emboss, etc. Universal drive stands may have cross-direction adjust capability such that the MUO position can be adjusted forward/backward at least +/- 10 mm around process center. Universal drive stands may enable drive to both rolls for servo-cam as needed. Universal drive stands may have optional (and CD-adjustable) vertical motor mounts; this is optional if MUO direct drive option is preferred.

Extra input/output ports may be mounted to the underside of the park bench apparatus for ease of input/output of data to Ethernet devices and logic controllers (high speed inputs/outputs, digital/analog in/outputs, etc).

### Modular Unit Operations for a Flexible Mount Converter

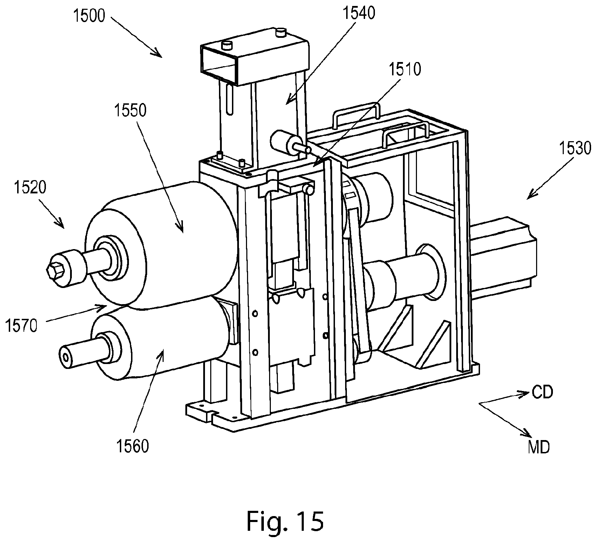

Modular unit operations designed for the flexible mount converter discussed above are shown in FIGS. 15-22. The new modular unit operation ("MUO") is a simple, spatially-effective, and process-efficient means for imparting rotary transformations onto passing high-speed webs. The modular unit operations comprise a rectangular frame and a unit operation, such as a roll. The frame is narrower in the machine direction than the diameter of at least one roll therein. This enables close proximity positioning (aka "roll-to-roll" positioning). MUOs are preferably standardized, configurable, re-usable, re-configurable, and stackable. Referring back to FIGS. 3 and 4, FIG. 3 shows a traditional converter layout utilizing non-modular unit operations at typical proximities. FIG. 4 shows an exemplary new converter layout utilizing modular unit operations at close proximities. The MUOs can be close coupled and arranged in various configurations on a flexible mount converter or other suitable converter. In preferred embodiments, MUOs are close coupled such that the distance between adjacent MUOs is less than 1m, or less than 0.8m, or less than 0.6m, or less than 0.4m, or less than 0.2m, or less than 0.1m, or less than 0.05m. In preferred embodiments, MUOs are close coupled without additional devices such as idlers, conveyors, or driven rolls between MUOs. In some embodiments, devices may be located on horizontal members above or below the park bench/MUOs. MUOs are like functional LEGO™ building blocks of the resulting converter. They can be mounted/located in any configuration/orientation. They enable significant reduction in converter space/footprint versus traditional, large, fully surrounding frame designs. MUOs can be horizontal, vertical, or angled to minimize footprint and optimize process efficiency. The MUOs may be stackable with one another or with one or more z-direction spacers to raise MUOs on a converter as needed to enable functional close coupling between MUOs. MUOs may be stacked two to five units high. MUOs may extend above the park bench, below the park bench, to either side of the park bench (drive side or operator side), or combinations thereof. MUOs can be used on-line (on the converter) or off-line as needed. MUOs are easily adjustable and re-configurable in both the MD and CD on the converter. MUOs can be located anywhere along the MD length of the converter. The MUOs set a new bar for standardization of unit operations that also enable flexibility. There is potential for high precision or low cost MUO designs.

Typically, the drive is direct mounted to the backside / drive-side of the MUO. There is a zero gear-in option for drives and a dual servo-cam option for each roll. Tooling on an MUO is quick to change without removing the MUO. MUOs may comprise features such as a smaller MD frame than a traditional unit op, pulleys fully enclosed on the drive side, through-bore slip rings, quick multi-connects, etc.

The new converter design may include cantilever (open-frame) style modular unit operations (see, *e.g.,* FIGS. 15-19) and/or frame-enclosed modular unit operations (see, *e.g.,* FIGS. 20-22) so long as they are designed for re-use for multiple process transformations, easily located in place, easily driven, and easily re-configurable. These modular frame unit operations are the building blocks of the converter. They can be located in any orientation. They can enable reduction in converter footprint versus traditional unit ops and layouts when placed in close proximity to one another. A first MUO and a second MUO are configurable such that the downstream side of the first MUO is close coupled with the upstream side of the second MUO. Preferably, the first and second MUOs are capable of imparting different transformations to a web. In some embodiments, each MUO comprises at least one roll as part of the tooling. A first roll from a first MUO may be close coupled with a second roll from a second MUO.

The MUO comprises a length in the machine direction, a width in the cross direction, and a height in the z-direction. In certain embodiments, the width is at least twice the length. Or, the width may be at least 1.5 times, 2 times, 3 times, or 4 times greater than the length. In certain embodiments, the height is at least twice the length. Or, the height may be at least 1.5 times, 2 times, 3 times, or 4 times greater than the length.

FIG. 15 shows an exemplary cantilever MUO 1500 for a flexible mount converter. The cantilever MUO comprises a load-bearing portion 1510, a transformation portion 1520, and a drive portion 1530. The transformation portion and drive portion are cantilevered from the load-bearing portion; these three portions are aligned in the cross direction. The load-bearing portion (in the case of a cantilever MUO) or modular frame (in the case of a non-cantilever MUO) comprises a load-generating device 1540, an operator side, a drive side opposing the operator side, an upstream side, a downstream side opposing the upstream side, and a bottom side which is joinable to a converter. The upstream side and downstream side are generally flat such that they are able to be close coupled with additional, similar modular unit operations. Load-bearing portions (or modular frames) are common and desirably 100% re-usable (only purchased for initial configuration then re-used for subsequent tooling, etc. changes). This enables lower capital costs and less environmental impact. Load-bearing portions (or modular frames) can have heat, chill, vacuum, compressed air, and trim removal capabilities. Load-bearing portions (or modular frames) can enable single or multi-lane configuration and enable product made in the alpha (*e.g.,* when the long length of the product is in the machine direction), beta (*e.g.,* when the long length of the product is in the cross direction), or combinations and hybrids of each direction.

The transformation portion comprises tooling capable of imparting a transformation to a web. In the case of a cantilever MUO, the transformation portion is joined to the operator side of the load-bearing portion. In the case of a non-cantilever MUO, the transformation portion is at least partially integrated with the modular frame or located within the modular frame. Simple cantilever MUOs may be mounted from either the operator side or the drive side of the load-bearing portion. This enables access to tooling from nearly any direction for, *e.g.,* ease of maintenance and operability. FIG. 15 shows tooling in the form of two rolls 1550, 1560 which mate at a nip 1570 to transform a passing web.

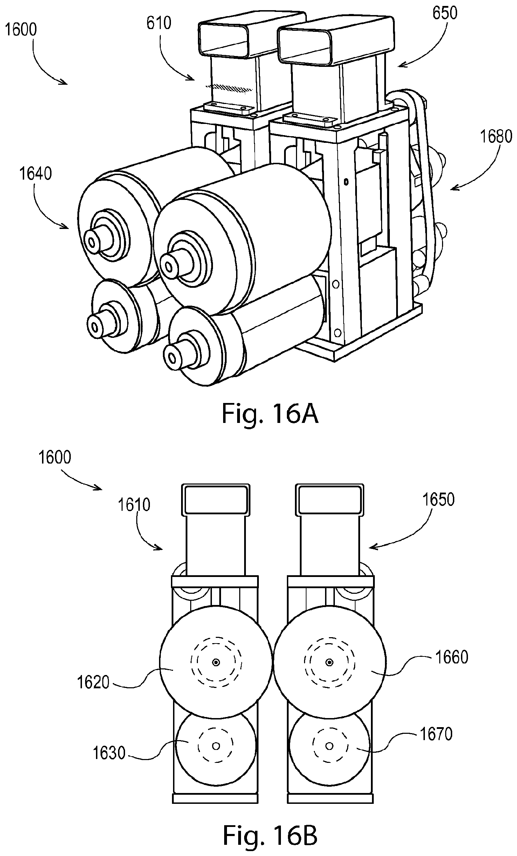

In one embodiment, the transformation portion tooling comprises at least one ring roll with an outer diameter. The transformation portion has a length in the machine direction, and the load-bearing portion (or modular frame) has a length in the machine direction. In some embodiments, the transformation portion length is the same as or greater than the load-bearing portion (or modular frame) length. For instance, the load-bearing portion (or modular frame) can be narrower in the machine direction than the outer diameter of the roll to optimize space utilization AND enable minimal free web spans. This facilitates 'roll-to-roll' web hand-offs between an array of unit operations 1600, such as depicted in FIGS. 16A and 16B. There are multiple ways to set the center-to-center spacing of the rolls, such as keeping the bottom roll stationary and allowing the top roll to be adjusted.

FIGS. 16A and 16B show an array 1600 of two exemplary close-coupled cantilever MUOs 1610, 1650. FIG. 16B is a front, or operator, side view of the array of two exemplary close-coupled cantilever modular unit operations, showing the transformation portions. The array 1600 demonstrates a roll-to-roll close-coupled orientation: MUO 1610 comprises two rolls 1620, 1630 and MUO 1650 comprises two rolls 1660, 1670. The transformation portion 1640 is preferably an operation selected from the group consisting of cutting, activation, crimping, embossing, aperturing, punching, bonding, and combinations thereof. Preferably, the transformation portion tooling is removable. For example, removable tooling can yield "blank slate" shafts that can be configured for many different uses. Tooling is configured through roll changes and ancillary kit changes. Roll changes and ancillary changes are executed via a minimal number of fasteners and simple tools for rapid change. One roll may be moveable in a linear direction (perpendicular to the roll axis) away from the other roll via the load-generating device 1540; this can aid in accessibility for tooling changes, rapid change overs, maintenance, etc. The rolls may be micro-adjusted (e.g., one full revolution of an adjustment knob can move a roll 0.5 mm) in the axial direction, one roll relative to another roll, to achieve a desired tooling setup necessary to the transformation, cross-sectional roll registration, or the like. The transformation portion tooling may be rapidly removable, such as with a single bolt removal wherein once the bolt is loosened, the transformation portion tooling may slide in the axial direction towards the operator side.

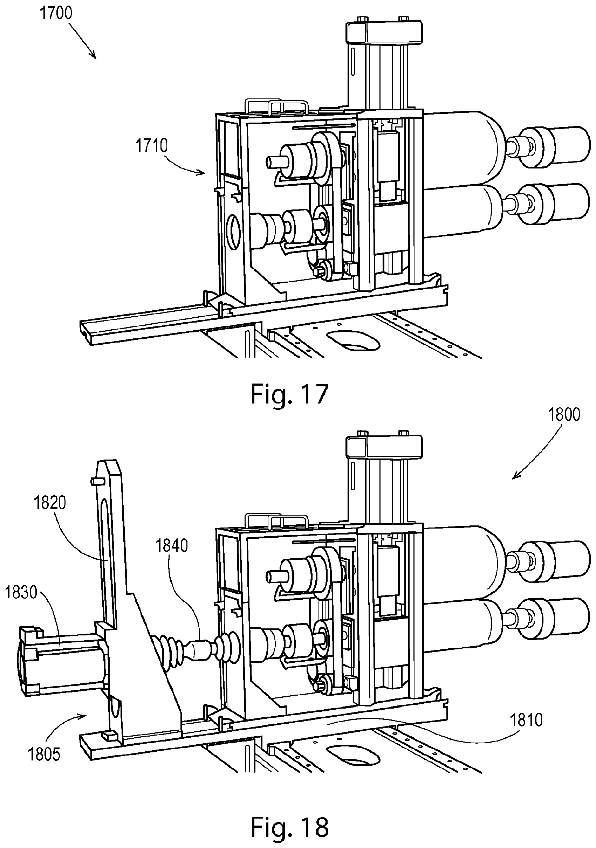

The drive portion 1680 is joined to the drive side of the load-bearing portion (in the case of a cantilever MUO) or modular frame (in the case of a non-cantilever MUO). In a preferred embodiment, the drive portion is joined without belts and pulleys. Generally, the tooling comprises at least two rolls. Accordingly, the drive portion may contain direct coupled servo-drives which may be configured for each roll individually such that the two rolls may be driven independently. This allows not only the roll speeds to be mismatched, but also, the speeds can be cammed. Each roll may be independently servo-cammed to enable 'one roll fits multiple' pitch lengths. Or, the rolls may be driven by a single motor. The motor may be directly connected to one roll, which in turn moves the other roll, via, for example, a belt. In some embodiments, the motor may be indirectly connected to one or more of the rolls, wherein the rolls are driven by the motor via a belt for example. FIG. 17 depicts a simple, cantilever MUO 1700 with a simple, direct drive 1710. In a preferred embodiment, the servo motor is direct mounted to the drive side of the load-bearing portion (or modular frame) with no gear-in. The RPM of the motor matches the RPM of the unit operation driven roll. Motors may be mounted on and supported by a universal drive stand. The universal drive stand is typically in the form of a vertical bracket located a distance from the load-bearing portion (or modular frame) in the cross direction. FIG. 18 illustrates a MUO 1800 having a universal drive stand 1805 comprising a base 1810, a vertical motor mount 1820, a motor 1830, and constant velocity style jackshaft 1840. The universal drive stand will enable cross directional adjustment via a hex adjustment on the front. The universal drive stand can drive different MUOs in different ways; direct couple as shown, vertical (external) motor mount with coupling, and vertical (external) motor mount through a 'constant velocity' jackshaft which allows for vertical movement of the shaft (allows for significant misalignment). The universal drive stand may support any and all unit operation styles and may also enable direct servo-camming of two rolls (upper and lower) through separate motors/drives. FIG. 19 illustrates an exemplary cantilever MUO 1910 on a park bench 1920; cantilever gusset brackets 1930 are present.

The drive portion comprises at least one plug 'n play feature selected from the group consisting of power, controls, utilities, and combinations thereof. In some embodiments, the plug 'n play feature is made through a single coupling.

In the case of a non-cantilever modular unit operation, much of the above disclosure also applies (e.g., MUOs may enable roll-to-roll web handoffs). FIG. 20 shows a schematic of an array 2000 of three frame-enclosed unit operations 2010, 2020, 2030 and one z-direction spacer 2040 on a pair of rails 2050. The MUO 2030 comprises two rolls 2060, 2070 in the transformation portion, a modular frame 2080, and a motor 2090 in the drive portion. FIGS. 21 and 22 depict non-cantilevered MUOs 2100, 2200. In FIG. 21, the rolls 2110, 2120 do not extend past the frame 2130, whereas in FIG. 22, one of the rolls 2210 extends wider than the frame 2230.

### Power, Controls, and Information Systems for a Flexible Mount Converter

The term "power" as used herein refers to the main electrical feeders going to a converter and powering devices. Power feeds may be AC or DC and low voltage or high voltage; a single MUO could have multiple power feeds.

The term "controls" as used herein refers to means for starting, stopping, adjusting set points, controlling end results, or controlling other functional aspects of devices via pneumatics, relays, solid-state relays, logic controllers, devices themselves, human-machine interfaces, or the like. Controls can be located in a panel or on a MUO. Controls can be physical controls or logical controls.

The term "information systems" as used herein refers to methods for storing and retrieving information from the converter or providing information to the converter. Exemplary methods are parameter recipe loads, human-machine interfaces, supervisory control and data acquisition ("SCADA") systems (*e.g.*, meeting spec limits or control limits), line event data systems (*e.g.*, reliability, other statistical data), time-stamped tag data, chart recorders, scope meters, data collection devices, etc.

The term "device" as used herein refers to any individual component associated with a flexible mount converter that serves a specific function, such as servo motors, push buttons, sensors, operator controls, heaters, pneumatic valves and actuators, metering rolls, idlers, printers, vision systems, driven rolls, web guides, or the like. One or more devices may be part of a modular unit operation, or one or more devices may be stand alone.

The term "plug-and-play" as used herein, refers to the ability to quickly and easily configure the function or location of devices as needed by the process or converter. For example, a given motor may be used today for an unwind spindle and tomorrow to drive a modular unit operation. Similarly, a given valve may be used today to load/unload a unit op, but tomorrow, the valve may be used to blow dust off of a sensor. In addition, plug-and-play allows us to choose which motor, valve, or device we are using at any given time, for example, by providing available connections to numerous devices.

Exemplary power, controls, and information systems designed for the flexible mount converter discussed above are shown in FIGS. 23-29. The flexible mount converter ("FMC") described herein comprises components such as a base, e.g. park bench, having a rail mount system that enables placement of a plurality of modular unit operations ("MUOs") in nearly any location imaginable. Just as the other components of the FMC have been designed to streamline the converter, so have the power, controls, and information systems ("PC&IS"). Specifically, the PC&IS are combined into a backbone infrastructure generally independent from the base features of the FMC (*e.g.,* the park bench apparatus and the MUOs). This approach decreases costs and provides improved agility. The FMC can be designed for ease of obsolescence; for instance, as PC&IS hardware and capability evolve, the ideal FMC will enable evolution through managed upgrades in existing panels versus large re-designs and replacements. For example, if a drive is improved, it can be swapped with the old drive that was in a panel rather than completely revamping or replacing the entire panel. One way PC&IS re-configurability may be improved is via smart plug-and-play features, meaning that additional functionality may be designed into an FMC for future use.

The FMC comprises a PC&IS framework comprising one or more repeatable PC&IS backbones having built-in PC&IS capabilities. Basically, each park bench apparatus may have its own PC&IS backbone, for example, one 5 m long FMC may comprise two 2.5 m long park benches and two associated 2.5 m long PC&IS backbones. As an analogy, the PC&IS framework is like a power strip that can accommodate multiple MUOs and other devices (for example, a vision inspection and/or rejection system). For instance, one 5 m long PC&IS framework may accommodate a device density of 16 MUO drives, 16 omega drives, 2 trim choppers, 4 glue systems, 8 heated zones, and 8 web guides. The PC&IS backbone may enable full converter pneumatics capability, *e.g.,* per 5 m of converter length. The PC&IS backbone may utilize a 'billboard approach' for plugged connections, meaning that all the drives are pre-wired to a plug connector that any appropriately sized motor can be connected to any appropriately sized drive. All plug connectors for a panel are located in a central location for easy access. The PC&IS backbone may utilize plug boards to enable wiring from servos to a billboard under the panel; cables may be added or removed as needed.

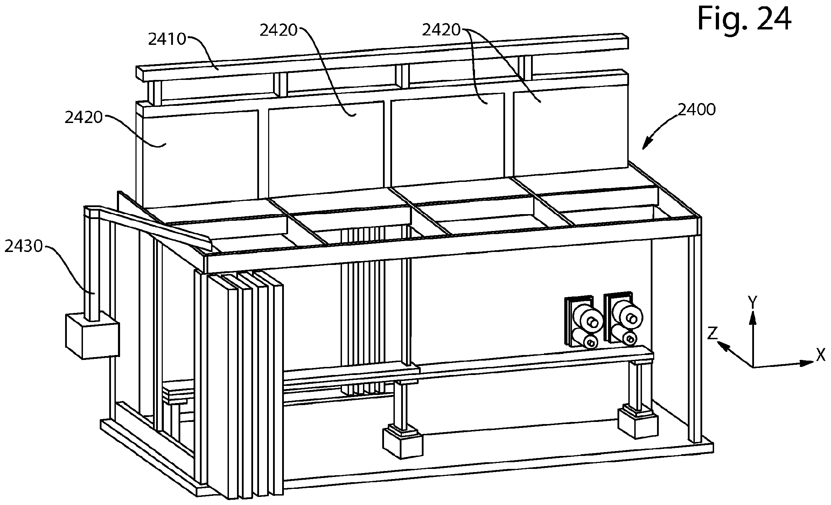

PC&IS may also be located in one or more panels along the converter. In one embodiment, overhead PC&IS panels 2320 are located on the top and/or rear portions of the surrounding frame 2330 to increase access or open area around the MUOs or decrease the footprint of the converter 2300, such as shown in FIG. 23. This minimizes cable lengths and complex cable runs, and can minimize the footprint of space utilization on the converter floor. In one embodiment, electrical panels are located on the top, drive side of the guarding. This enables significant space savings on the plant floor without building a complete mezzanine support system. FIGS. 8A through 8D and illustrate such panels. In a preferred embodiment, there are two panels for each horizontal rail segment, so that, for instance, a park bench apparatus comprising two 2.5m segments (providing a 5 m converter length) will have four panels. PC&IS devices may be located within each 2.5 m park bench section, however, cables can cross for a 'mix-and-match' scenario. Controls can reach outside of the converter for ancillaries (for example, a drive in the 5m section may be used to power unwind spindles outside of the 5 m section). As shown in FIG. 23, one additional power distribution panel 2310 (and optional logic controller) may be provided per converter 2300; this panel may be located on the drive side of converter. Roll-around platforms may be used for access (e.g., portable stairs). As shown in FIG. 24, ductwork for cables 2410 may be present adjacent to panels 2420 on a converter 2400. FIG. 24 also shows an exemplary (human machine interface) HMI 2430 for line control. Cable baskets may be located under panels and around sides of the PC&IS framework for convenient routing of cables.

MUOs and/or park benches may comprise at least one plug-and-play feature selected from the group consisting of power, controls, information systems, and combinations thereof. The features are typically located in the drive portion. The plug-and-play feature may be made through a single coupling. For instance, a single multi-connector may be used with MUOs to enable ease of re-configuring MUOs, rapid changeovers, etc. A single coupling or connector may allow operators to quickly connect/disconnect MUOs without needing to plug in sensors, lubrication, air lines, etc.

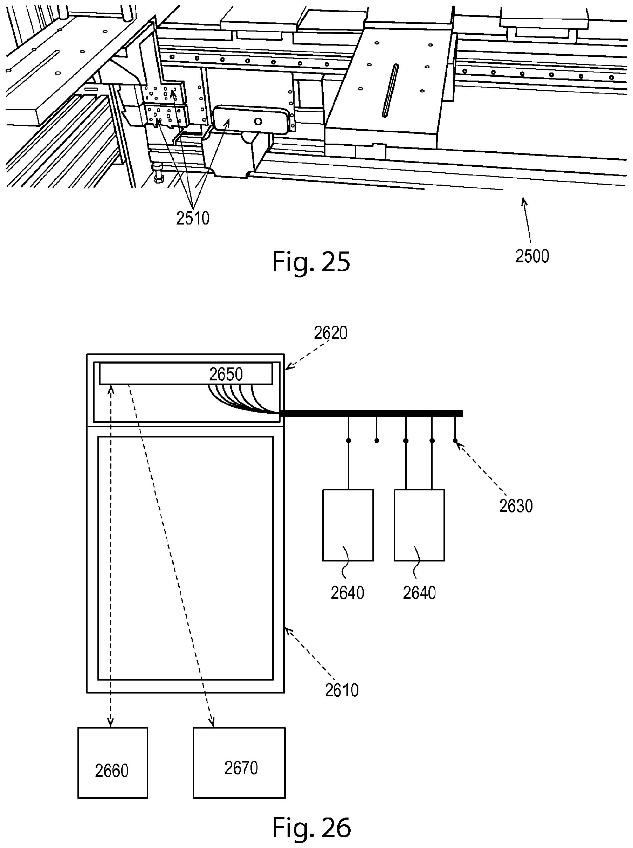

The park bench may utilize plug and play PC&IS with, for example, all drives and servo motors remaining with the park bench and utilized as needed via a simple, configurable HMI. Adequate drive density, power, and input/output ("I/O") may be installed then re-configured with minimal effort as necessary. The park bench 2500 may comprise I/O blocks 2510 for digital analog and high speed I/O as shown in FIG. 25. A 5 m long PC&IS framework may comprise 32 digital inputs, 32 digital outputs, 32 programmable limit switch inputs, 32 programmable limit switch outputs, 32 analog inputs, 16 analog outputs, 12 temperature controlled zones, etc.

The PC&IS backbone may comprise vision systems, inspection, and data collection capabilities (*e.g.,* a National Instruments rack run directly to Ethernet switch already on the FMC).

The PC&IS are preferably Ethernet-based and enable full on-board diagnostics and condition monitoring. This permits more universal use: devices/applications and their connections may be simplified and reduce construction effort. In a preferred embodiment, only Ethernet protocol devices are used (e.g., no other networks such as SERCOS™, ControlNet™, DeviceNet™, Profibus™ and the like). Because it is all on Ethernet, going back to the logic controller, pneumatic and motion control solutions are easily integrated, construction efforts and material costs are minimized and future expansion is easier. There may be a managed Ethernet switch for fast Ethernet connection. In some embodiments, SERCOS™, ControlNet™, DeviceNet™, Profibus™, Ethernet™, the like or combinations thereof may be utilized.

Convenience Ethernet outlets may be spaced throughout the converter length to enable cameras, programming, new devices, or the like. Extra input/output ports may be mounted to the underside of the park bench apparatus for ease of input/output of data to Ethernet devices and logic controllers (high speed inputs/outputs, digital/analog in/outputs, etc). The PC&IS may use recipe-download-based changeovers, for instance, to minimize manual adjust points.

Software may be designed and/or programmed using PackML standards. PackML comprises a common programming structure, consistent mode and state definitions which drive a common look and feel among equipment. Preferably, the PC&IS backbone incorporates only one or two processors (or less than six) versus the traditional eight or nine processors. Code (*e.g.,* PackML) has series of parallel rungs and can activate relevant one(s) as needed - ignoring code that isn't currently being used (which takes up memory but not processing speed) and thereby needing fewer processors than a traditional converting line. All converter states may be defined (e.g. stopped, stopping, ramping, running, faulted, resetting, rejecting and the like) to simplify coding time of the logic controller memory. Via plug-and-play, drive density may be determined and designed to meet future needs with extra capability built in. For instance, eight unit operation motors/servos may be supplied for each repeating horizontal segment (e.g., 2.5m segment) along the FMC length. Likewise, eight metering motors/servos may be supplied for each repeating horizontal segment along the FMC length. Wires are preferably coiled up and ready for use; plugboards with permanent terminations may be present in panels coming to the boards. Wires may be used as needed (motor cables + encoder cables). Electrical background and conditionally-dependent control software are independent of the particular converter/MUO design. Rather, the PC&IS backbone is a flexible infrastructure that can make multiple products. Almost any drive/motor combo can be used for almost any function, *e.g*., metering rolls, unit ops, unwind.

The PC&IS backbone may comprise plug-and-play pneumatics. A pneumatics bus with tubing within it, connected to a valve system & manifold, may be pre-routed and pre-labeled for use when needed. Manual regulators and gauges are no longer needed for each valve on the converter. Instead, the pneumatics may be adjusted via human-machine interface ("HMI") for instance, on a dedicated pneumatic digital display panel (such as the pneumatic control panel 2340 shown in FIG. 23). An accessible valve manifold may be mounted flush with, for example, a drive-side guard door for ease of troubleshooting without disconnecting motion/Air bus or opening guards. One or more Ethernet valve manifolds may enable on-board monitoring and diagnostics as well as on-board automatic control for optimizing efficiency and minimizing maintenance (*e.g.,* closed-loop, open-loop, or feed-forward control), and also minimize wiring and construction efforts to install. One or more mobile diagnostic user interfaces may enable easy troubleshooting. One 5m long PC&IS framework may comprise the following pneumatics capabilities: 16 load/unload pressure regulators (including 12 double-acting cylinders (each having 4 valve options) and 4 spring-return), 16 blow-off valves (including 16 pressure regulators), 16 sensor block connections (including 4 pressure regulators), and 24 vacuum sensors. For all of the valves, set points are logic-based and virtually (without regulators) changed via the HMI based on the state of the line. While this virtual change is possible, gauges may still be useful for monitoring.

As shown in FIGS. 26-28, the PC&IS backbone may comprise a quick-connect valve coupling system allowing an operator to use a valve that is conveniently coupled to a connection located near a modular unit operation. The quick-connect valve system may run the length of the converter to allow for multiple access points. The quick-connect system may be located on the top or bottom of the converter. There is one quick-connect fitting per valve. Each valve is configurable depending on the type of air needed. A short hose can run from the valve system to modular unit operations.

FIG. 26 depicts a drive side guard door 2610 with a stationary pneumatic panel 2620 mounted on it. Pneumatic connections, one connection per valve, are consolidated and brought to the converter, where quick-connect fittings 2630 exist to easily connect pneumatic tubing to the MUOs 2640. The valve bank 2650 comprises solenoid valves, air connections, pressure sensors, proportional pressure regulators, and other I/O functions. The valve bank communicates via Ethernet with the HMI 2660, and transmits data wirelessly to a mobile diagnostic user interface 2670 with no adjustment capability. Pressure is adjusted by the user at the HMI or via recipe download, and is controlled by the proportional pressure valves located on the valve bank.

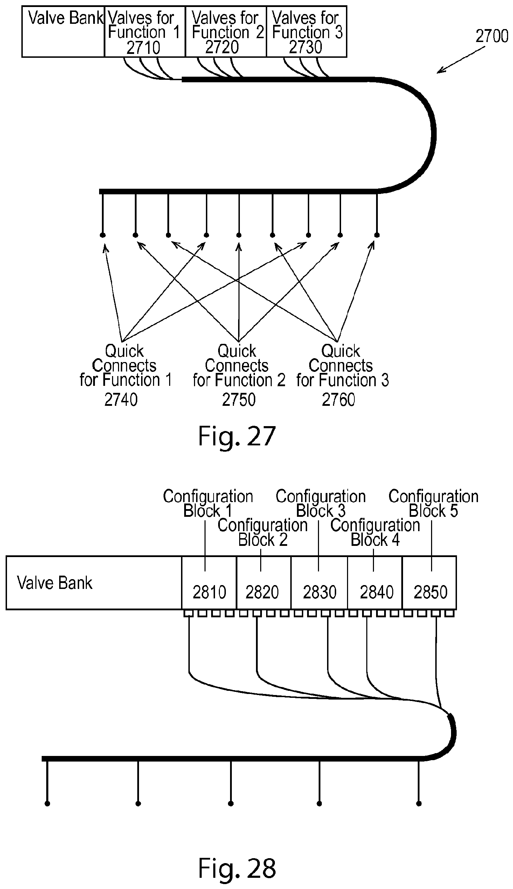

As illustrated in FIG. 27, the valve system 2700 may comprise an assortment of valves 2710, 2720, 2730 having different functions, allowing the user to choose a quick-connect port 2740, 2750, 2760 associated with the desired valve. For instance, a first section may have reject valves, a second section may have blow-off valves, a third section may have load/unload valves, etc.

As illustrated in FIG. 28, a valve block 2810, 2820, 2830, 2840, 2850 which previously had one specific function that was not easily changeable may now be user-configurable (for example, in the form of configuration blocks) for several different functions (e.g., it may be a sensor blow-off or a load-unload valve or a reject valve) and easily changeable between functions.

The valve system may comprise a combination of the two above-described options, meaning that some of the valves may be changeable and others may be selected from options already present. The valve manifold may have Ethernet communications for control, diagnostic, and condition monitoring.

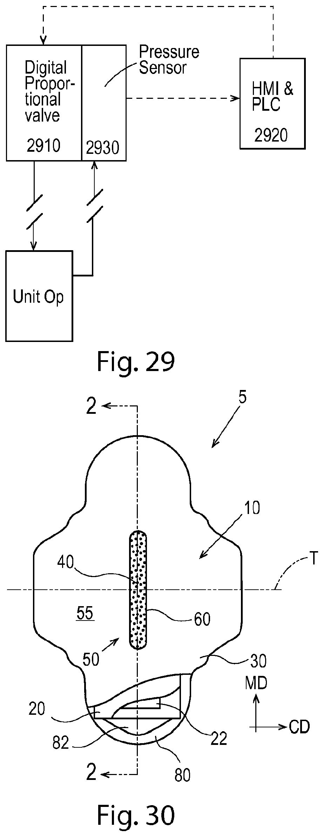

As shown in FIG. 29, digital proportional valves (aka servo-pressure valves) 2910 may be used in place of traditional on/off valves with regulators. Digital proportional valves can be integrated in such a way as to enable remote control of air pressure from the HMI 2920 or recipe. In addition to digital proportional valves, simple on/off valves (e.g., five-way, two-position) may be utilized for load and unload applications. Preferably, the use of logic based pressure solutions is maximized. Digital proportional valves, when combined with pressure sensors 2930, enable closed-loop control of pressure, as well as predictive and automatic maintenance adjustments.

The PC&IS backbone may comprise plug-and-play motors/servo drives. Motors/servo drives may either drive small rolls and conveyors or drive MUOs and unwinds. The motors/servo drives may drive the largest unit ops on the FMC, which may in turn drive the smaller units as well. There may be enough of each size of motor and servo drive to operate all known units and rolls on the FMC. One or more, or all, servo drives may be wired with quick connect plugs which can reach anywhere along the length of the park bench (regardless of whether there is a motor/unit operation present or not). As MUOs are moved on/off the park bench apparatus, the operator can plug in the size of motor needed, and configure on the HMI. Each motor/servo system can run in multiple modes, for example, pitched unit op, geared unit op/conveyor, unwind/rewind, cammed, registered. An operator may change/control these settings via HMI or changes can be made via recipe download.

The PC&IS backbone may comprise plug-and-play sensors. Along the park bench there may be quick connect input/output ("I/O") inlands/connection points that connect digital, analog, and high speed inputs and outputs (e.g., PLS I/O) to the logic controller. As individual inputs and outputs (e.g., proximity sensors, photoeyes, limit switches, camera strobe, lighting, valves, etc.) are needed on the converter they can be plugged into the I/O islands which are located conveniently along the park bench. Some or all of these I/O islands may be Ethernet modules which may enable on-board control, monitoring and diagnostics and reduces wiring and construction effort. These can be configured by the operator via the HMI or via a recipe download to allow the I/O to perform specific functions (e.g., line stops, registration, product count, feature detection, product detection, and the like).

The PC&IS backbone may comprise plug-and-play heaters. Along the park bench, there may be quick-connect heater/RTD plugs which connect the control for heaters and temperature feedback to the logic controller. Each connection may provide power and signal feedback for one heated zone (typically one heated unit op roll). The quick-connect plugs may be spaced along the park bench conveniently for access where MUOs are installed. For instance, there may be four heated zones per X m converter length or per X m rail segment length.

The PC&IS backbone may comprise plug-and-play cooling. Along the park bench, there may be quick connect RTD plugs which connect the control for analog valves for chilling coolant control and temperature feedback to the logic controller. Each connection may provide power and signal feedback for one chilled zone (typically one chilled unit op roll). The quick-connect plugs may be spaced along the park bench conveniently for access where MUOs are installed.

The PC&IS backbone may comprise one or more of the above-described features.

### Methods for Manufacturing Absorbent Articles on a Flexible Mount Converter

Absorbent articles, such as feminine hygiene articles, may be manufactured on the flexible mount converter described herein. The flexible mount converter may comprise various combinations of elements selected from park benches, cantilever modular unit operations, non-cantilever modular unit operations, complete access guarding, virtual mirror plates, power, controls, information systems, etc. First, a web is provided to the converting line. Then, a first modular unit operation imparts a first transformation to the web. Then, a second modular unit operation may impart a second transformation to the web. In most cases, the second transformation is different from the first transformation.

### General Description of an Absorbent Article

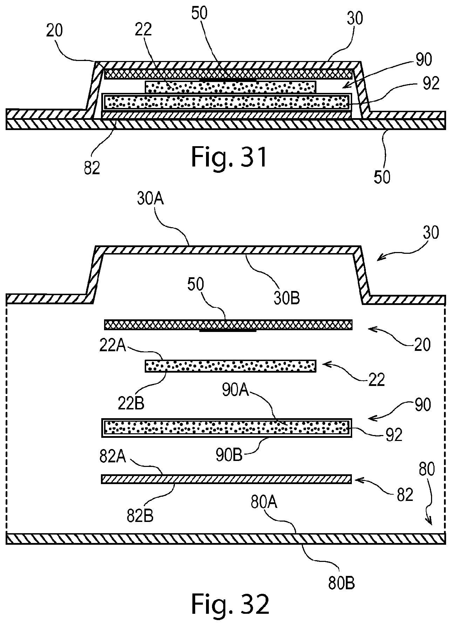

An exemplary absorbent article 5 according to the present disclosure, shown in the form of a feminine hygiene article such as sanitary napkin or incontinence pad, is represented in FIGS. 30-32. This type of absorbent article is shown for illustration purpose only as the present disclosure can be used for making a wide variety of other absorbent articles. FIG. 30 is a top view of the example absorbent article 5, in a flat-out state, with portions of the structure being cut-away to more clearly show the construction of the absorbent article 5. FIG. 31 is a cross-sectional view of the absorbent article of FIG. 30 taken along line 2-2, while FIG. 32 is an exploded cross-sectional view of the absorbent article of FIG. 31.

Referring to FIG. 30, the absorbent article 5 can have a substantially planar configuration and a centroid 40. The centroid 40 is the in-plane center of mass of the absorbent article 5. The centroid 40 is at the intersection between the longitudinal centerline L and transverse centerline T. The transverse centerline T is orthogonal to the longitudinal centerline L. The absorbent article 5 can, but need not be, symmetric about the transverse centerline T. The absorbent article 5 has a body-facing surface 10 and a garment facing surface (not shown).

The absorbent article 5 comprises a plurality of layers to promote certain liquid handling behaviors. Example layers include a liquid-permeable topsheet 30 and an absorbent core 90. The topsheet may be a film-nonwoven composite, such as a polyethylene film-polyethylene nonwoven composite. Some embodiments can also include a top core 22, as illustrated. The absorbent core 90 can have a number of suitable arrangements, for example the absorbent core 90 can have a tissue outer wrapping 92 (FIG. 31). The absorbent articles can also have a backing material 82 and a backsheet 80.

To help ensure that fluids flow into the absorbent core 90, some absorbent articles are constructed with what is sometimes referred to as a secondary topsheet 20 ("STS") positioned intermediate the topsheet 30 and the absorbent core 90. This secondary topsheet 20 is designed to acquire the fluid on the liquid-permeable topsheet 30 and distribute it to the underlying absorbent core 90. To help ensure that the secondary topsheet 20 transfers the fluid to the absorbent core 90, the secondary topsheet 20 can have sufficient capillarity to draw the fluid through the liquid-permeable topsheet 30. To ensure that the fluid flow continues onto the absorbent core 90, the secondary topsheet 20 can be designed with more permeability than the absorbent core 90, and less capillarity than the absorbent core 90. For example, a secondary topsheet can be an airlaid-tissue web made from hydrophilic cellulosic fibers and polyethylene powder, sometimes referred to as an airlaid STS. Or, a secondary topsheet can be a spunlace web. A spunlace web may be a hydroentangled fibrous structure with a basis weight between about 35 grams per square meter (gsm) and about 85 gsm. The spunlace web may comprise about 30% to about 60%, by weight, of cellulosic fibers, about 5% to about 30%, by weight, of non-cellulosic fibers, and about 30% to about 55%, by weight, of polyolefin-based binder fibers. In one embodiment, the topsheet is a film-nonwoven composite, such as a polyethylene film-polyethylene nonwoven composite, and the STS is a spunlace web.

The substrates may comprise any suitable woven, nonwoven, film, combination or laminate of any of the foregoing materials. Non-limiting examples of suitable substrates include cellulose; a mixture of cellulosic fibers and polyethylene or polyethylene-polypropylene bicomponent fibers or particulate; nonwoven substrates, such as for example, polypropylene nonwoven; polyethylene film; bi-component nonwoven or film; polyethylene terephthalate nonwoven or film; films, such as polymeric or thermoplastic films; foils, such as metallic foils (e.g. aluminum, brass, copper, and the like); webs comprising sustainable polymers; foams; fibrous nonwoven webs comprising synthetic fibers (*e.g*. TYVEK®); collagen films; chitosan films; rayon; cellophane; and the like. Suitable webs further include laminates or blends of these materials. Suitable films include both cast and blown. Exemplary thermoplastic films suitable for use as the second substrate are low density polyethylene ("LDPE"), linear low-density polyethylene ("LLDPE"), and blends of LLDPE and LDPE. Films may be apertured. In some embodiments, the substrates may have a basis weight of about 6 gsm to about 100 gsm. Other types of substrates can be sandwiched in between two layers of nonwovens or films.

Substrates can also optionally include colorants, such as pigment, lake, toner, dye, ink or other agent used to impart a color to a material, to improve the visual appearance of a substrates or the resultant laminate. Suitable pigments herein include inorganic pigments, pearlescent pigments, interference pigments, and the like. Non-limiting examples of suitable pigments include talc, mica, magnesium carbonate, calcium carbonate, magnesium silicate, aluminum magnesium silicate, silica, titanium dioxide, zinc oxide, red iron oxide, yellow iron oxide, black iron oxide, carbon black, ultramarine, polyethylene powder, methacrylate powder, polystyrene powder, silk powder, crystalline cellulose, starch, titanated mica, iron oxide titanated mica, bismuth oxychloride, and the like. Suitable colored webs are described in US 2010/0233438 and US 2010/0233439.

Although the apparatuses and methods will be described in the context of the feminine hygiene article 5 shown in FIGS. 30-32, it is to be appreciated that the methods and apparatuses herein may be used to manufacture various absorbent articles, such as for example, diapers or diaper pants.

While the present invention is described from a viewpoint of fabricating absorbent articles, the present invention also applies to other industries / purposes / product types (e.g., printing, bag making, pharmaceutical packing lines, support bandages, pessaries, etc.). Any manufacturing process, especially those already based on modules, may be improved by using one or more of the components described herein. For instance, a printing process may involve a park bench and modular unit operations, wherein every color is a separate MUO.

Similarly, while a 5m long park bench and/or converter is used as an example multiple times throughout the detailed description, it is not meant to be limiting. It is to be understood that the exact lengths depend on the type of converting line, product being manufactured, size of unit operations, etc. So, for instance, the length may be 1m to 10m, or even outside that range.

Embodiments are contemplated where a cantilever modular unit operation for a flexible mount converter comprises a machine direction, a cross direction, and a vertical z-direction. The cantilevered modular unit operation also includes a load-bearing portion comprising: a load-generating device; an operator side; a drive side opposing the operator side; an upstream side; a downstream side opposing the upstream side; and a bottom side which is joinable to the converter; a transformation portion joined to the operator side of the load-bearing portion, comprising tooling capable of imparting a transformation to a web; and a drive portion joined to the drive side of the load-bearing portion, wherein the drive portion is joined without belts and pulleys. And, the transformation portion and drive portion are cantilevered from the load-bearing portion.

In some embodiments, the drive portion may further comprise at least one plug 'n play feature selected from the group consisting of power, controls, utilities, and combinations thereof. The plug 'n play feature can be achieved through a single coupling. The upstream side and downstream side can be generally flat such that they are able to be close coupled with additional, similar modular unit operations. In some embodiments, the transformation portion has a length in the machine direction, wherein the load-bearing portion has a length in the machine direction, and wherein the transformation portion length is greater than the load-bearing portion length. In some embodiments, the transformation portion tooling comprises a roll with an outer diameter, and wherein the load-bearing portion is narrower in the machine direction than the outer diameter of the roll. In some embodiments, the transformation portion performs an operation selected from the group consisting of cutting, activation, crimping, bonding, embossing, aperturing, punching, and combinations thereof. In some embodiments, the transformation portion tooling is removable. In some embodiments, the tooling may comprise at least two rolls, wherein at least two rolls are driven independently. In some embodiments, the tooling comprises at least two rolls, wherein at least one roll is moveable in a linear direction away from the other roll via the load-generating device. In some embodiments, the modular unit operation further comprises a z-direction spacer. In some embodiments, the modular unit operation comprises a length in the machine direction, a width in the cross direction, and a height in the z-direction; wherein the width is at least twice the length. In some embodiments, the cantilever modular unit operation of further comprises a length in the machine direction, a width in the cross direction, and a height in the z-direction; wherein the height is at least twice the length.

In some embodiments, a flexible mount converter comprises the cantilever modular unit operation and further comprising a second modular unit operation. The second modular unit operation comprises, in some embodiments, a modular frame comprising: a load-generating device; an operator side, a drive side opposing the operator side, an upstream side, a downstream side opposing the upstream side, a bottom side which is joinable to the converter; and a transformation portion comprising tooling that is capable of imparting a transformation to a web. The second modular unit operation further comprises a drive portion joined to the drive side of the modular frame, wherein the drive portion is joined without belts and pulleys. The first modular unit operation and the second modular unit operation may be configurable such that the downstream side of the first modular unit operation is close coupled with the upstream side of the second modular unit operation, the first modular unit operation and second, cantilever unit operation are capable of imparting different transformations to a web.

In some embodiments, an array of two or more cantilever modular unit operations for a flexible mount converter may be utilized. The array comprises a first cantilever modular unit operation and a second cantilever modular unit operation. The first modular unit operation comprises a first load-bearing portion comprising: a load-generating device; an operator side; a drive side opposing the operator side; an upstream side; a downstream side opposing the upstream side; and a bottom side which is joinable to the converter. The first cantilever modular unit operation further comprises a first transformation portion joined to the operator side of the first load-bearing portion, comprising tooling capable of imparting a first transformation to a web, and a first drive portion joined to the drive side of the first load-bearing portion, wherein the first drive portion is joined without belts and pulleys. The first transformation portion and first drive portion are cantilevered from the first load-bearing portion. The second cantilever modular unit operation comprises a second load-bearing portion comprising: a load-generating device; an operator side; a drive side opposing the operator side; an upstream side; a downstream side opposing the upstream side; and a bottom side which is joinable to the converter. The second cantilever modular unit operation further comprises a second transformation portion joined to the operator side of the second load-bearing portion, comprising tooling capable of imparting a second transformation to a web, and a second drive portion joined to the drive side of the second load-bearing portion, wherein the second drive portion is joined without belts and pulleys. The second transformation portion and second drive portion are cantilevered from the second load-bearing portion, and the second transformation is different from the first transformation. The downstream side of the first cantilever modular unit operation is close coupled with the upstream side of the second cantilever modular unit operation, and the first and second cantilever modular unit operations are re-configurable on the converter.

In some embodiments, the array further comprises a z-direction spacer. In some embodiments, the first transformation portion tooling comprises a first roll, wherein the second transformation portion tooling comprises a second roll, and wherein the first roll and the second roll are close coupled. In some embodiments, the first and second modular unit operations are stackable.

A method of manufacturing absorbent articles, in some embodiments, comprises the steps of: providing a web; providing a first cantilever modular unit operation, and imparting a first transformation onto the web. The first cantilever modular unit having a load-bearing portion comprising: a load-generating device; an operator side; a drive side opposing the operator side; an upstream side; a downstream side opposing the upstream side; and a bottom side which is joinable to the converter. The first cantilever modular unit further having a transformation portion joined to the operator side of the load-bearing portion, comprising tooling capable of imparting a transformation to a web, and a drive portion joined to the drive side of the load-bearing portion, wherein the drive portion is joined without belts and pulleys. The transformation portion and drive portion are cantilevered from the load-bearing portion.

A flexible mount converter comprising a machine direction length and a cross direction width, a power, controls, and information systems backbone, in some embodiments, comprises: one or more electrical feeders capable of supplying alternating current, direct current, or both; one or more controls selected from the group consisting of: pneumatics, relays, solid-state relays, logic controllers, devices, human-machine interfaces, or combinations thereof; and methods for storing and retrieving information from the converter or providing information to the converter selected from the group consisting of: parameter recipe loads, human-machine interfaces, supervisory control and data acquisition systems, line event data systems, time-stamped tag data, chart recorders, scope meters, data collection devices, or combinations thereof. The backbone is physically integrated in a plug-and-play manner with one or more components of the converter, the components selected from the group consisting of: a park bench apparatus having a horizontal rail mount system, a modular unit operation removeably mountable to the park bench apparatus, complete access guarding, and combinations thereof. In some embodiments, the converter comprises complete access guarding comprising a surrounding frame, and wherein the backbone comprises one or more panels located on top of the surrounding frame. In some embodiments, the backbone further comprises one or more plug-and-play features selected from the group consisting of pneumatics, vacuums, sensors, heaters, cooling, motors/servo drives, digital proportional valves, and combinations thereof. In some embodiments, the backbone further comprises software designed to PackML standards. In some embodiments, the backbone further comprises data input and/or data output ports. In some embodiments, the backbone further comprises vision system, inspection, and data collection capabilities. In some embodiments, the backbone further comprises a pneumatic quick-connect valve coupling system. In some embodiments, the quick-connect valve system provides multiple access points along the length of the converter.

A flexible mount converter comprising a machine direction, a cross direction, a drive side, an operator side, a top side, a left end, a right end, a complete access guard system. The complete access guard system may comprise a surrounding frame; a horizontal track located in the machine direction at about ground level along the operator side of the converter; a series of foldable guard doors having a top and a bottom, wherein the top is joined to the surrounding frame and the bottom is joined to the horizontal track; wherein the foldable guard doors are capable of sliding towards the left end or the right end of the converter; and one or more drive panels located on top of the surrounding frame. The converter may be accessed from both the operator side and the drive side, and the converter is able to manufacture and/or assemble various components of absorbent articles. In some embodiments, the flexible mount converter may comprise additional guarding located on a side of the flexible mount converter selected from the group consisting of operator side, drive side, top side, bottom side, left end, right end, or combination thereof. In some embodiments, the complete access guarding system further comprises: a second horizontal track located in the machine direction at about ground level along the drive side of the converter; and a second series of foldable guard doors having a top and a bottom, wherein the top is joined to the surrounding frame and the bottom is joined to the second horizontal track; wherein the second foldable guard doors are capable of sliding towards the left end or the right end of the converter. In some embodiments, the complete access guarding system further comprises electrical panels located on top of the surrounding frame. In some embodiments, the complete access guarding system further comprises keyless guard door switches. In some embodiments, the complete access guarding system further comprises clear polycarbonate guarding on the top side of the flexible mount converter. In some embodiments, the complete access guarding system further comprises a virtual programmable logic controller to control the flexible mount converter via Ethernet. In some embodiments, there is a lack of vertical posts between the left end and right end of the converter.

## Claims

1. A flexible mount converter for fabricating absorbent articles (5), **characterized in that** the flexible mount converter comprises:
a. a machine direction, a cross direction, a drive side, an operator side, a top side, a left end, and a right end;
b. a length in the machine direction and a width in the cross direction;
c. a drive side and an operator side;
d. a base (900) supporting a horizontal rail mount system (960);
e. two or more modular unit operations (830, 840, 1500, 1610, 1650, 1700, 1800, 1910, 2030, 2100, 2200, 2640) removeably mountable to the horizontal rail mount system in a close-coupled manner, such that a distance between adjacent modular unit operations is less than 1 m;
f. complete access guarding (860); and
g. a virtual mirror plate formed of a plurality of plates selected from the group consisting of scab plates, barrier plates, or combinations thereof, wherein the virtual mirror plates are supported by vertical and/or horizontal members attached to the flexible mount converter;
wherein the flexible mount converter is capable of producing an absorbent article that comprises the web.

2. The flexible mount converter of Claim 1, further comprising vertical posts (880), wherein the vertical posts are spaced at least 4m apart along the machine direction length of the flexible mount converter.

3. The flexible mount converter of Claim 1, wherein the virtual mirror plate comprises a scab plate having a device mounted thereon.

4. The flexible mount converter of any of the preceding claims, wherein the modular unit operations comprise combinations of cantilevered modular unit operations and non-cantilevered modular unit operations.

5. The flexible mount converter of any of the preceding claims, wherein the converter is 5m or shorter in length, and wherein the flexible mount converter comprises 8 or more modular unit operations.

6. The flexible mount converter of any of the preceding claims, wherein the flexible mount converter has a line speed of greater than 6 m/s.

7. The flexible mount converter of any of the preceding claims, further comprising a device selected from the group consisting of metering rolls, idlers, printers, vision systems, driven rolls, web guides, and combinations thereof.

8. The flexible mount converter of any of the preceding claims, further comprising at least one comprise at least one plug-and-play feature selected from the group consisting of power, controls, information systems, and combinations thereof.

9. The flexible mount converter of any one of Claims 1-3 and 5-8, wherein the modular unit operations are cantilevered modular unit operations.

10. The flexible mount converter of any one of Claims 1-3, and 5-8, wherein the modular unit operations are non-cantilevered modular unit operations.

## Patentansprüche

1. Konverter mit elastischer Halterung zum Herstellen von Absorptionsartikeln (5), **dadurch gekennzeichnet, dass**
der Konverter mit elastischer Halterung umfasst:
a. eine Maschinenlaufrichtung, eine Querrichtung, eine Antriebsseite, eine Bedienerseite, eine Oberseite, ein linkes Ende und ein rechtes Ende;
b. eine Länge in der Maschinenlaufrichtung und eine Breite in der Querrichtung;
c. eine Antriebsseite und eine Bedienerseite;
d. eine Basis (900), die ein horizontales Schienenhalterungssystem (960) trägt;
e. zwei oder mehr modulare Grundoperationen (830, 840, 1500, 1610, 1650, 1700, 1800, 1910, 2030, 2100, 2200, 2640), die abnehmbar am horizontalen Schienenhalterungssystem in einer eng gekoppelten Weise montierbar sind, sodass ein Abstand zwischen benachbarten modularen Grundoperationen weniger als 1 m beträgt;
f. vollständige Zugangssicherung (860); und
g. eine virtuelle Spiegelplatte, gebildet aus einer Vielzahl von Platten, ausgewählt aus der Gruppe, bestehend aus Schuppenplatten, Barriereplatten oder Kombinationen davon, wobei die virtuellen Spiegelplatten von vertikalen und/oder horizontalen Elementen gestützt werden, die am Konverter mit elastischer Halterung befestigt sind;
wobei der Konverter mit elastischer Halterung dazu geeignet ist, einen Absorptionsartikel zu produzieren, der die Bahn umfasst.

2. Konverter mit elastischer Halterung nach Anspruch 1, der ferner vertikale Ständer (880) umfasst, wobei die vertikalen Ständer entlang der Maschinenlaufrichtungslänge des Konverters mit elastischer Halterung mindestens 4 m voneinander beabstandet sind.

3. Konverter mit elastischer Halterung nach Anspruch 1, wobei die virtuelle Spiegelplatte eine Schuppenplatte mit einer daran montierten Vorrichtung umfasst.

4. Konverter mit elastischer Halterung nach einem der vorstehenden Ansprüche, wobei die modularen Grundoperationen Kombinationen freitragender modularer Grundoperationen und nicht-freitragender modularer Grundoperationen umfassen.

5. Konverter mit elastischer Halterung nach einem der vorstehenden Ansprüche, wobei der Konverter eine Länge von 5 m oder kürzer aufweist und wobei der Konverter mit elastischer Halterung 8 oder mehr modulare Grundoperation umfasst.

6. Konverter mit elastischer Halterung nach einem der vorstehenden Ansprüche, wobei der Konverter mit elastischer Halterung eine Bandgeschwindigkeit von mehr als 6 m/s aufweist.

7. Konverter mit elastischer Halterung nach einem der vorstehenden Ansprüche, ferner umfassend eine Vorrichtung, ausgewählt aus der Gruppe, bestehend aus Dosierwalzen, Leitwalzen, Druckern, Sichtsystemen, angetriebenen Walzen, Bahnführungen und Kombinationen davon.

8. Konverter mit elastischer Halterung nach einem der vorstehenden Ansprüche, ferner umfassend mindestens ein Plug-and-Play-Merkmal, ausgewählt aus der Gruppe, bestehend aus Energie, Steuerungen, Informationssystemen und Kombinationen davon.

9. Konverter mit elastischer Halterung nach einem der Ansprüche 1-3 und 5-8, wobei die modularen Grundoperationen freitragende modulare Grundoperationen sind.

10. Konverter mit elastischer Halterung nach einem der Ansprüche 1-3 und 5-8, wobei die modularen Grundoperationen nicht-freitragende modulare Grundoperation sind.

## Revendications

1. Convertisseur de support souple pour la fabrication d'articles absorbants (5), **caractérisé en ce que**
le convertisseur de support souple comprend :
a. un sens machine, un sens travers, un côté entraînement, un côté opérateur, un côté supérieur, une extrémité gauche et une extrémité droite ;
b. une longueur dans le sens machine et une largeur dans le sens travers ;
c. un côté entraînement et un côté opérateur ;
d. une base (900) supportant un système de support de rail horizontal (960) ;
e. deux opérations unitaires modulaires (830, 840, 1500, 1610, 1650, 1700, 1800, 1910, 2030, 2100, 2200, 2640) ou plus pouvant être montées de manière amovible sur le système de support de rail horizontal d'une manière à couplage étroit, de telle sorte qu'une distance entre des opérations unitaires modulaires adjacentes est inférieure à 1 m ;
f. une sécurisation d'accès complète (860) ; et
g. une plaque de miroir virtuel formée d'une pluralité de plaques choisies dans le groupe constitué de plaques de raccordement, plaques de barrière, ou combinaisons de celles-ci, où les plaques de miroir virtuel sont supportées par des éléments verticaux et/ou horizontaux fixés au convertisseur de support souple ;
où le convertisseur de support souple est capable de produire un article absorbant qui comprend la bande.

2. Convertisseur de support souple selon la revendication 1, comprenant en outre des montants verticaux (880), dans lequel les montants verticaux sont espacés d'au moins 4 m le long de la longueur du sens machine du convertisseur de support souple.

3. Convertisseur de support souple selon la revendication 1, dans lequel la plaque de miroir virtuel comprend une plaque de raccordement ayant un dispositif monté sur celle-ci.

4. Convertisseur de support souple selon l'une quelconque des revendications précédentes, dans lequel les opérations unitaires modulaires comprennent des combinaisons d'opérations unitaires modulaires en porte-à-faux et d'opérations unitaires modulaires non en porte-à-faux.

5. Convertisseur de support souple selon l'une quelconque des revendications précédentes, où le convertisseur fait 5 m de long ou moins, et où le convertisseur de support souple comprend 8 opérations unitaires modulaires ou plus,

6. Convertisseur de support souple selon l'une quelconque des revendications précédentes, où le convertisseur de support souple a une vitesse de ligne supérieure à 6 m/s.

7. Convertisseur de support souple selon l'une quelconque des revendications précédentes, comprenant en outre un dispositif choisi dans le groupe constitué de rouleaux doseurs, galets, imprimantes, systèmes de vision, rouleaux entraînés, guides de bande, et leurs combinaisons.

8. Convertisseur de support souple selon l'une quelconque des revendications précédentes, comprenant en outre au moins une caractéristique prête à l'emploi choisie dans le groupe constitué de puissance, commandes, systèmes d'information, et leurs combinaisons.

9. Convertisseur de support souple selon l'une quelconque des revendications 1 à 3 et 5 à 8, dans lequel les opérations unitaires modulaires sont des opérations unitaires modulaires en porte-à-faux.

10. Convertisseur de support souple selon l'une quelconque des revendications 1 à 3 et 5 à 8, dans lequel les opérations unitaires modulaires sont des opérations unitaires modulaires non en porte-à-faux.
